# EUROPEAN PATENT APPLICATION

(11) **EP 1 880 994 A1**
(43) Date of publication of application: **23.01.2008**
(21) Application number: 06746268.9
(22) Date of filing: 11.05.2006
(51) Int. Cl.: C07D 405/14, A61K 31/454, A61P 1/08, A61P 1/14, A61P 7/10, A61P 7/12, A61P 13/00, A61P 15/00, A61P 25/06, A61P 25/18, A61P 25/20, A61P 25/22, A61P 25/28, A61P 25/30, A61P 25/34, A61P 29/00, A61P 43/00

(54) **CRYSTAL OF INDOLE DERIVATIVE HAVING PIPERIDINE RING AND PROCESS FOR PRODUCTION THEREOF**

(30) Priority: 11.05.2005 JP 2005008632; 11.05.2005 US 126209; 10.11.2005 JP 2005325712
(71) Applicant: Eisai R&D Management Co., Ltd., Tokyo 112-8088 (JP)
(72) Inventor: SAKAGUCHI, Takahisa,c/o Tsukuba Research Lab., Tsukuba-shi, Ibaraki 300-2635 (JP); SUZUKI, Yuichi, c/o Tsukuba Research Laboratory, Tsukuba-shi, Ibaraki 300-2635 (JP); ITO, Koichi, c/o Tsukuba Research Laboratory, Tsukuba-shi, Ibaraki 300-2635 (JP); NIIJIMA, Jun, c/o Tsukuba Research Laboratory, Tsukuba-shi, Ibaraki 300-2635 (JP); MIYAZAWA, Mamoru, Kamisu-shi, Ibaraki 314-0255 (JP); SHIMIZU, Toshikazu, Kamisu-shi, Ibaraki 314-0255 (JP); GOTODA, Masaharu, c/o Tsukuba Research Laboratory, Tsukuba-shi, Ibaraki 300-2635 (JP); SUZUKI, Naoko, Ibaraki 300-1233 (JP); HASEBE, Takashi, Andover, Massachusetts 0181-2447 (US)
(74) Representative: HOFFMANN EITLE
(86) International application number: PCT/JP2006/309459
(87) International publication number: WO 2006/121104

(57) **Abstract**

A crystal of 1-[1-[2-(7-methoxy-2,2-dimethyl-4-oxochroman-8-yl)-ethyl]piperidin-4-yl]-N-methyl-1H-indole-6-carboxamide fumarate which has peaks at chemical shifts of about 124.0 ppm and about 26.8 ppm in a ¹³C solid NMR spectrum.

## Description

### TECHNICAL FIELD

The present invention relates to a crystal form of 1-{1-[2-(7-methoxy-2,2-dimethyl-4-oxochroman-8-yl)-ethyl]piperidin-4-yl}-*N*-methyl-1*H*-indole-6-carboxamide that has 5-HT_{1A} receptor antagonistic effect and binding effect, which is useful as a preventive agent or therapeutic agent for lower urinary tract symptoms, and particularly, urinary storage symptoms. The present invention also relates to a process for producing said crystal.

### BACKGROUND ART

The 5-HT_{1A} receptor is one serotonin receptors. Compounds having 5-HT_{1A} receptor antagonistic effect and binding affinity are expected as a preventive agent or therapeutic agent for depression, anxiety disorders,cognitive impairment and urinary disturbance. Examples of such a compound include various previously-reported compounds which have a piperidine ring (refer to Patent Document 1, Patent Document 2 and Patent Document 3).

Patent Document 1: WO99/06348
Patent Document 2: JP-A-2002-114684
Patent Document 3: WO98/43956

### DISCLOSURE OF THE INVENTION

The present inventors discovered, as a novel piperidine-ring-containing indole derivative having 5-HT_{1A} receptor antagonistic effect and binding affinity, the compound represented by the following general formula (I), 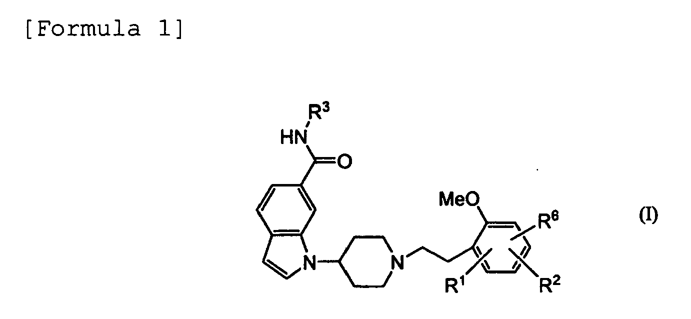 wherein R¹ and R² are substituents adjacent to each other, and together with two carbon atoms to each of which they attach, form:
(1) a 5- to 7-membered non-aromatic carbocyclic group,
(2) a 5- to 7-membered non-aromatic heterocyclic group,
(3) a 6-membered aromatic carbocyclic group, or
(4) a 5- or 6-membered aromatic heterocyclic group,
   which may be substituted by 1 to 4 substituents selected from the following substituent group B1;
   R³ represents a hydrogen atom or a methyl group; and
   R⁶ represents a substituent selected from the following substituent group A1,
   Substituent group A1: (1) a hydrogen atom, (2) a halogen atom, (3) a cyano group, (4) a hydroxyl group, (5) a nitro group, (6) a carboxyl group, (7) a C3-C8 cycloalkyl group, (8) a C2-C6 alkenyl group, (9) a C2-C6 alkynyl group, (10) a C1-C6 alkylthio group, (11) a C1-C6 alkoxycarbonyl group, (12) a C1-C6 alkylsulfonyl group, (13) a C1-C6 alkyl group (wherein the C1-C6 alkyl group may be substituted by 1 to 3 substituents selected from the group consisting of a halogen atom, a hydroxyl group and a C1-C6 alkoxy group), (14) a C1-C6 alkoxy group (wherein the C1-C6 alkoxy group may be substituted by 1 to 3 halogen atoms), (15) an amino group (wherein the amino group may be substituted by a substituent selected from the group consisting of a C1-C6 alkyl group, a formyl group, a C1-C6 alkanoyl group and a C1-C6 alkylsulfonyl group) and (16) a carbamoyl group (wherein the carbamoyl group may be substituted by one or two C1-C6 alkyl groups),
   Substituent group B1: (1) a hydrogen atom, (2) a halogen atom, (3) a cyano group, (4) a hydroxyl group,
(5) a nitro group, (6) an oxo group, (7) a carboxyl group, (8) a C3-C8 cycloalkyl group, (9) a C2-C6 alkenyl group, (10) a C2-C6 alkynyl group, (11) a C1-C6 alkylthio group, (12) a C1-C6 alkoxycarbonyl group, (13) a C1-C6 alkylsulfonyl group, (14) a C1-C6 alkyl group (wherein the C1-C6 alkyl group may be substituted by a halogen atom, a hydroxyl group and a C1-C6 alkoxy group), (15) a C1-C6 alkoxy group (wherein the C1-C6 alkoxy group may be substituted by 1 to 3 halogen atoms), (16) an amino group (wherein the amino group may be substituted by a substituent selected from the group consisting of a C1-C6 alkyl group, a formyl group, a C1-C6 alkanoyl group and a C1-C6 alkylsulfonyl group), (17) a carbamoyl group (wherein the carbamoyl group may be substituted by one or two C1-C6 alkyl groups), (18) a C1-C6 alkoxyimino group, (19) a C5-C6 cycloalkyl group formed by two C1-C3 alkyl groups attaching to the same carbon atom and (20) a tetrahydropyranyl group formed by two C1-C3 alkyl groups attaching to the same carbon atom, together with an oxygen atom and the carbon atom. Patent applications have already been filed for this compound (International Patent Application No. PCT/JP 2005/008632 and U.S. Patent Application No. 11/126209). This compound exhibits 5-HT_{1A} receptor antagonistic action and binding affinity, and is useful as a preventive agent or therapeutic agent for lower urinary tract symptoms, and particularly, urinary storage symptoms.

Especially, the compound 1-{1-[2-(7-methoxy-2,2-dimethyl-4-oxochroman-8-yl)-ethyl]piperidin-4-yl}-*N*-methyl-1*H*-indole-6-carboxamide represented by the following formula (i), which is included in the above-described general formula (I), 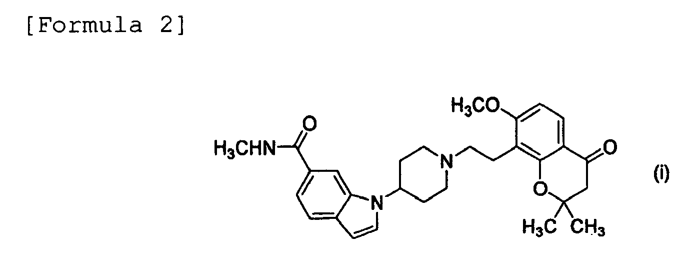 is expected as having an excellent effect.

On the other hand, in the case of using a compound which has crystalline polymorphs as a pharmaceutical, it is necessary to stably supply a compound having a uniform crystal form in order to ensure a uniform quality and a constant potency of action that are required as a pharmaceutical. Further, there is a need for a crystal form which is capable of maintaining the same quality during storage and drug formulation processes such as mixing and granulation. Accordingly, when the active ingredient of the drug is obtained as a crystalline substance, it is preferable for the substance to be composed of a single crystal form, to be stable and have good physical properties and to be free from impurities such as metals. In addition, there is also a need to develop a process capable of producing such a crystalline stably on an industrial scale.
Accordingly, it is an object of the present invention to provide a crystal form of fumarate or tartrate of the compound (i) represented by the above-described formula (i) and a production process thereof.

As a result of continued dynamic research, the present inventors discovered a crystal form of fumarate or tartrate of compound (i), and a production process thereof, thereby arriving at the present invention.
Specifically, the present invention relates to:
(1) A crystal form of 1-{1-[2-(7-methoxy-2,2-dimethyl-4-oxochroman-8-yl)-ethyl]piperidin-4-yl}-N-methyl-1*H*-indole-6-carboxamide fumarate;
(2) A crystal form (form A) of 1-{1-[2-(7-methoxy-2,2-dimethyl-4-oxochroman-8-yl)-ethyl]piperidin-4-yl}-*N*-methyl-1*H*-indole-6-carboxamide fumarate which has peaks at chemical shifts of about 124.0 ppm and about 26.8 ppm in a ¹³C solid NMR spectrum;
(3) A crystal form (form B) of 1-{1-[2-(7-methoxy-2, 2-dimethyl-4-oxochroman-8-yl)-ethyl]piperidin-4-yl}-*N*-methyl-1*H*-indole-6-carboxamide fumarate which has peaks at chemical shifts of about 143.8 ppm and about 32.8 ppm in a ¹³C solid NMR spectrum;
(4) A crystal form (form D) of 1-{1-[2-(7-methoxy-2,2-dimethyl-4-oxochroman-8-yl)-ethyl]piperidin-4-yl}-*N*-methyl-1*H*-indole-6-carboxamide fumarate which has peaks at chemical shifts of about 190.5 ppm and about 138.0 ppm in a ¹³C solid NMR spectrum;
(5) A crystal form (form A) of 1-{1-[2-(7-methoxy-2,2-dimethyl-4-oxochroman-8-yl)-ethyl]piperidin-4-yl}-*N*-methyl-1*H*-indole-6-carboxamide fumarate which has diffraction peaks at diffraction angles (2θ±0.2°) of 18.2° and 30.9° in X-ray powder diffraction;
(6) A crystal form (form B) of 1-{1-[2-(7-methoxy-2,2-dimethyl-4-oxochroman-8-yl)-ethyl]piperidin-4-yl}-*N*-methyl-1*H*-indole-6-carboxamide fumarate which has diffraction peaks at diffraction angles (2θ±0.2°) of 27.6° and 32.7° in X-ray powder diffraction;
(7) A crystal form (form C) of 1-{1-[2-(7-methoxy-2,2-dimethyl-4-oxochroman-8-yl)-ethyl]piperidin-4-yl}-*N*-methyl-1*H*-indole-6-carboxamide fumarate which has diffraction peaks at diffraction angles (2θ±0.2°) of 9.8° and 19.7° in X-ray powder diffraction;
(8) A crystal form (form D) of 1-{1-[2-(7-methoxy-2,2-dimethyl-4-oxochroman-8-yl)-ethyl]piperidin-4-yl}-*N*-methyl-1*H*-indole-6-carboxamide fumarate which has diffraction peaks at diffraction angles (2θ±0.2°) of 8.3° and 14.0° in X-ray powder diffraction;
(9) A process for producing the crystal form (form A) according to the above-described (2) or (5), comprising heating 1-{1-[2-(7-methoxy-2,2-dimethyl-4-oxochroman-8-yl)-ethyl]piperidin-4-yl}-*N*-methyl-1*H-*indole-6-carboxamide fumarate in a mixed solvent of acetone and water to dissolve it, then cooling the solution to precipital crystals and filtering off the crystals;
(10) A process for producing the crystal form (form B) according to the above-described (3) or (6), comprising heating 1-{1-[2-(7-methoxy-2,2-dimethyl-4-oxochroman-8-yl)-ethyl]piperidin-4-yl}-*N*-methyl-1*H-*indole-6-carboxamide fumarate in a mixed solvent of n-propanol and water to dissolve it, then cooling the solution to precipital crystals and filtering off the crystals;
(11) A process for producing the crystal form (form C) according to the above-described (7), comprising heating 1-{1-[2-(7-methoxy-2,2-dimethyl-4-oxochroman-8-yl)-ethyl]piperidin-4-yl}-*N*-methyl-1*H-*indole-6-carboxamide fumarate in a mixed solvent of methanol and water to dissolve it, then cooling the solution to precipital crystals and filtering off the crystals;
(12) A process for producing the crystal form (form D) according to the above-described (4) or (8), comprising heating 1-{1-[2-(7-methoxy-2,2-dimethyl-4-oxochroman-8-yl)-ethyl]piperidin-4-yl}-*N*-methyl-1*H-*indole-6-carboxamide fumarate in an alcohol solvent, an amide solvent, an ester solvent or a mixed solvent thereof to dissolve it, then cooling the solution to precipital crystals and filtering off the crystals;
(13) A crystal form of 1-{1-[2-(7-methoxy-2,2-dimethyl-4-oxochroman-8-yl)-ethyl]piperidin-4-yl}-N-methyl-1*H*-indole-6-carboxamide tartrate;
(14) A process for producing the crystal form according to the above-described (14), comprising dissolving 1-{1-[2-(7-methoxy-2,2-dimethyl-4-oxochroman-8-yl)-ethyl]piperidin-4-yl}-*N*-methyl-1*H-*indole-6-carboxamide tartrate in a mixed solvent of methanol and water, then removing the mixed solvent by distillation;
(15) A pharmaceutical composition comprising the crystal form according to any of the above-described (1) to (8) and (13) as an active ingredient;
(16) A preventive agent or therapeutic agent for lower urinary tract symptoms comprising the crystal according to any of the above-described (1) to (8) and (13) as an active ingredient;
(17) The agent according to the above-described (16), which is a preventive agent or therapeutic agent for urinary storage symptoms;
(18) The agent according to the above-described (16), which is a preventive agent or therapeutic agent for urinary frequency or urinary incontinence;
(19) A preventive agent or therapeutic agent for cognitive impairment associated with Alzheimer's disease or senile dementia, learning or memory disorder, or anxiety disorder, comprising the crystal form according to any of the above-described (1) to (8) and (13) as an active ingredient;
(20) A preventive agent or therapeutic agent for schizophrenia, emotional disorder, alcohol and/or cocaine dependence, nicotine addiction or symptoms associated with smoking cessation, or visual attention disorder, comprising the crystal form according to any of the above-described (1) to (8) and (13) as an active ingredient; and
(21) A preventive agent or therapeutic agent for sleep disorder, migraine, temperature instability, eating disorder, vomiting, gastrointestinal disorder, or sexual dysfunction, comprising the crystal form according to any of the above-described (1) to (8) and (13) as an active ingredient.

According to the present invention, compound (i) can be easily produced free from metals or other such impurities and in single crystal form on an industrial scale. The crystal according to the present invention exhibits good physical properties and is suitable for use as an active ingredient in a preventive agent or therapeutic agent for lower urinary tract symptoms.

### BRIEF DESCRIPTION OF THE DRAWINGS

Fig. 1 is the solid NMR spectrum of 1-{1-[2-(7-methoxy-2,2-dimethyl-4-oxochroman-8-yl)-ethyl]piperidin-4-yl}-*N*-methyl-1*H*-indole-6-carboxamide fumarate (form A);
Fig. 2 is the solid NMR spectrum of 1-{1-[2-(7-methoxy-2,2-dimethyl-4-oxochroman-8-yl)-ethyl]piperidin-4-yl}-*N*-methyl-1*H*-indole-6-carboxamide fumarate (form B);
Fig. 3 is the solid NMR spectrum of 1-{1-[2-(7-methoxy-2,2-dimethyl-4-oxochroman-8-yl)-ethyl]piperidin-4-yl}-*N*-methyl-1*H*-indole-6-carboxamide fumarate (form D);
Fig. 4 is the solid NMR spectrum of 1-{1-[2-(7-methoxy-2,2-dimethyl-4-oxochroman-8-yl)-ethyl]piperidin-4-yl}-*N*-methyl-1*H*-indole-6-carboxamide tartrate;

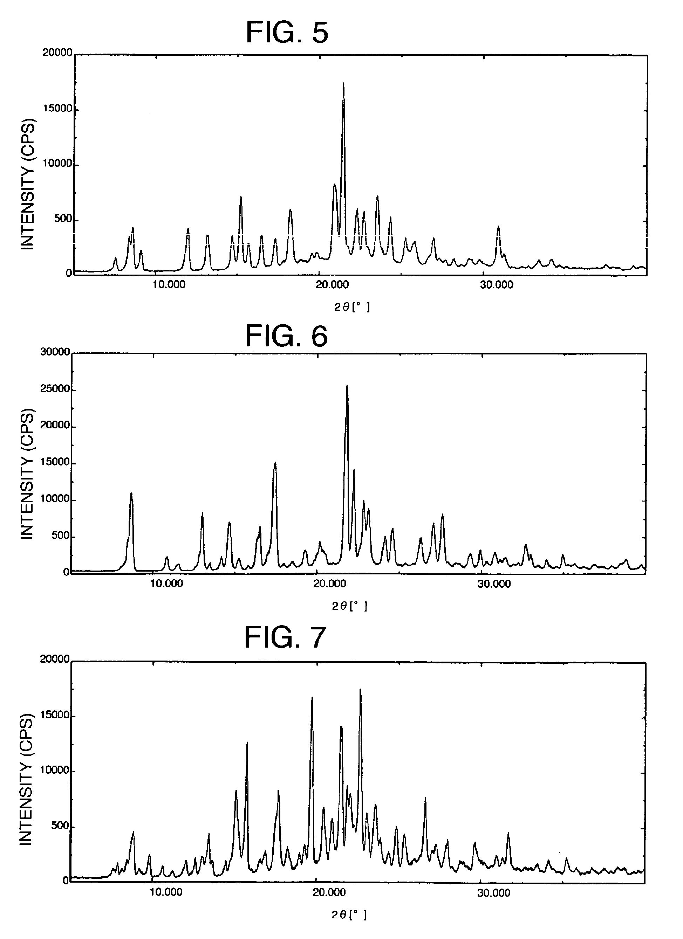

Fig. 5 is the X-ray powder diffraction pattern of 1-{1-[2-(7-methoxy-2,2-dimethyl-4-oxochroman-8-yl)-ethyl]piperidin-4-yl}-*N*-methyl-1*H-*indole-6-carboxamide fumarate (form A);
Fig. 6 is the X-ray powder diffraction pattern of 1-{1-[2-(7-methoxy-2,2-dimethyl-4-oxochroman-8-yl)-ethyl]piperidin-4-yl}-*N*-methyl-1*H-*indole-6-carboxamide fumarate (form B);
Fig. 7 is the X-ray powder diffraction pattern of 1-{1-[2-(7-methoxy-2,2-dimethyl-4-oxochroman-8-yl)-ethyl]piperidin-4-yl}-*N*-methyl-1*H-*indole-6-carboxamide fumarate (form C);

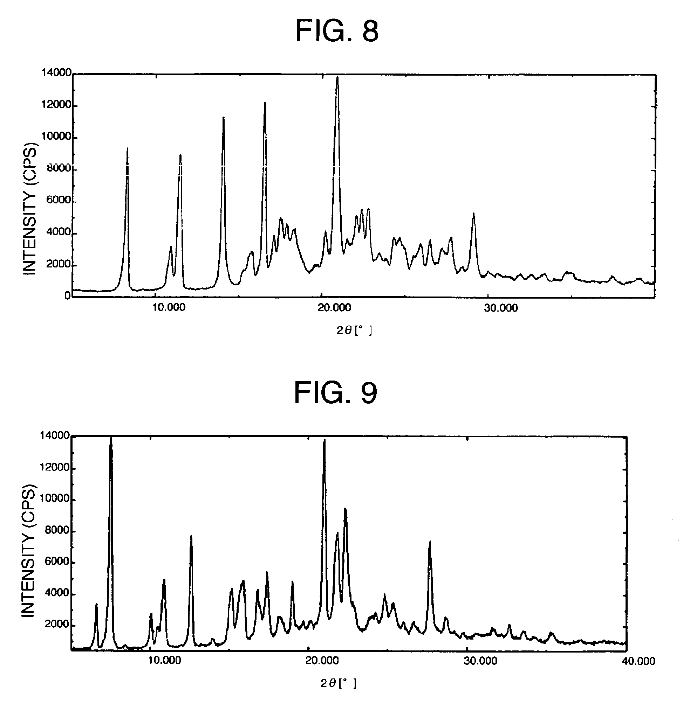

Fig. 8 is the X-ray powder diffraction pattern of 1-{1-[2-(7-methoxy-2,2-dimethyl-4-oxochroman-8-yl)-ethyl]piperidin-4-yl}-*N-*methyl-1*H-*indole-6-carboxamide fumarate (form D);
Fig. 9 is the X-ray powder diffraction pattern of 1-{1-[2-(7-methoxy-2,2-dimethyl-4-oxochroman-8-yl)-ethyl]piperidin-4-yl}-*N*-methyl-1*H-*indole-6-carboxamide tartrate;
Fig. 10 is the infrared spectra of a crystal form (form A) of 1-{1-[2-(7-methoxy-2,2-dimethyl-4-oxochroman-8-yl)-ethyl]piperidin-4-yl}-*N*-methyl-1*H-*indole-6-carboxamide fumarate;
Fig. 11 is the infrared spectra of a crystal form (form B) of 1-{1-[2-(7-methoxy-2,2-dimethyl-4-oxochroman-8-yl)-ethyl]piperidin-4-yl}-*N*-methyl-1*H-*indole-6-carboxamide fumarate;
Fig. 12 is the infrared spectra of a crystal form (form C) of 1-{1-[2-(7-methoxy-2,2-dimethyl-4-oxochroman-8-yl)-ethyl]piperidin-4-yl}-*N*-methyl-1*H-*indole-6-carboxamide fumarate;
Fig. 13 is the infrared spectra of a crystal form (form D) of 1-{1-[2-(7-methoxy-2,2-dimethyl-4-oxochroman-8-yl)-ethyl]piperidin-4-yl}-*N*-methyl-1*H-*indole-6-carboxamide fumarate;
Fig. 14 is the infrared spectra of a crystal form of 1-{1-[2-(7-methoxy-2,2-dimethyl-4-oxochroman-8-yl)-ethyl]piperidin-4-yl}-N-methyl-1*H*-indole-6-carboxamide tartrate;
Fig. 15 is the thermal analysis diagram of a crystal form (form A) of 1-{1-[2-(7-methoxy-2,2-dimethyl-4-oxochroman-8-yl)-ethyl]piperidin-4-yl}-N-methyl-1*H*-indole-6-carboxamide fumarate;
Fig. 16 is the thermal analysis diagram of a crystal form (form B) of 1-{1-[2-(7-methoxy-2,2-dimethyl-4-oxochroman-8-yl)-ethyl]piperidin-4-yl}-N-methyl-1*H*-indole-6-carboxamide fumarate;
Fig. 17 is the thermal analysis diagram of a crystal form (form C) of 1-{1-[2-(7-methoxy-2,2-dimethyl-4-oxochroman-8-yl)-ethyl]piperidin-4-yl}-N-methyl-1*H*-indole-6-carboxamide fumarate;
Fig. 18 is the thermal analysis diagram of a 1-{1-[2-(7-methoxy-2,2-dimethyl-4-oxochroman-8-yl)-ethyl]piperidin-4-yl}-*N*-methyl-1*H*-indole-6-carboxamide fumarate crystal (form D); and
Fig. 19 is the thermal analysis diagram of a crystal form of 1-{1-[2-(7-methoxy-2,2-dimethyl-4-oxochroman-8-yl)-ethyl]piperidin-4-yl}-*N*-methyl-1*H-*indole-6-carboxamide tartrate.

### BEST MODE FOR CARRYING OUT THE INVENTION

The present invention will now be described in more detail.
The physical property data, specifically solid NMR spectra, X-ray powder diffraction patterns, infrared absorption spectra and thermal analysis diagrams, of the forms A to D crystal form s of fumarate and the crystal form of tartrate of compound (i) are shown below.

### Solid NMR

### Measurement Conditions

Apparatus: AVANCE 400 MHz (Bruker, Switzerland)
Probe: 7 mm-CP/MAS (Bruker)
NMR Cell Diameter: 7 mm
Rotation Frequency: 6,000 round/sec
Integration Frequency: Fumarate form A 2,048 times, form B 1,954 times, form D 2,048 times and tartrate 1,024 times
Latency: 10 sec
Contact time: 5,000 microseconds
External Standard: Chemical shift at carbonyl-carbon glycine set at 176.03 ppm

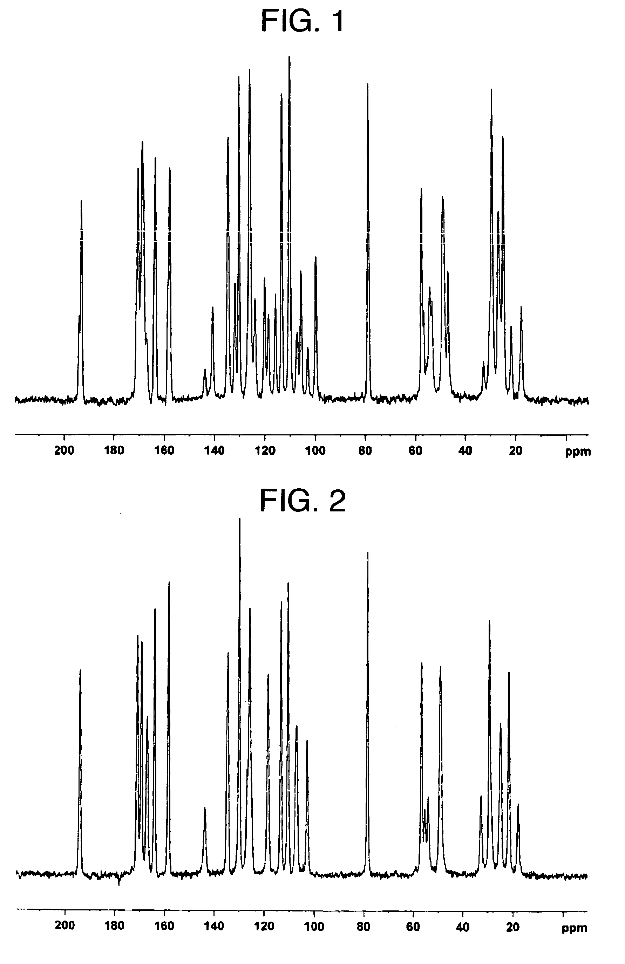

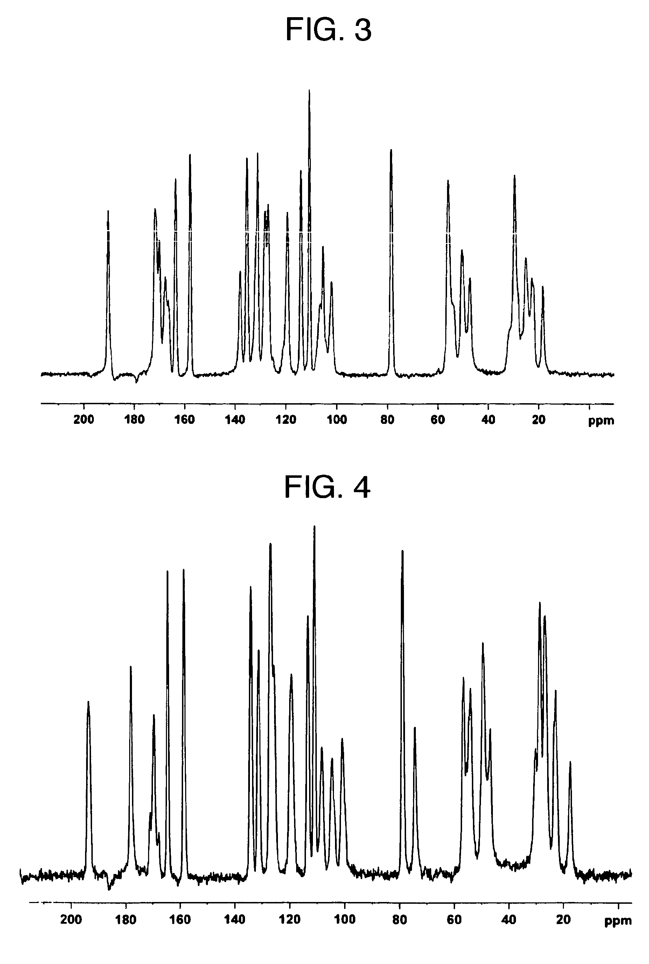

The solid NMR measurement results of the forms A, B and D crystal forms of fumarate and of the crystalline of tartrate are respectively shown in Figs. 1 to 4.

The peaks of the forms A, B and D crystal forms of fumarate and the crystal form of tartrate in solid NMR are shown in Table 1.

**[Table 1]**

| Form A (ppm) | Form B (ppm) | Form D (ppm) | Tartrate (ppm) |
|---|---|---|---|
| 193.0 | 194.1 | 190.5 | 193. 7 |
| 170.4 | 171.1 | 171.7 | 178.1 |
| 168.7 | 169.5 | 170.1 | 170.9 |
| 163.6 | 167.1 | 167.8 | 169.6 |
| 157.8 | 164.2 | 166.5 | 167.8 |
| 140.8 | 158.5 | 163.6 | 164.6 |
| 134.6 | 143.8 | 157.8 | 158.7 |
| 131.8 | 134.7 | 138.0 | 134.1 |
| 130.2 | 130. 1 | 135.3 | 131.2 |
| 125.9 | 126.8 | 131.1 | 126.9 |
| 124.0 | 126.0 | 128.1 | 125.7 |
| 120.1 | 118.7 | 126.9 | 119.4 |
| 118.6 | 113.5 | 119.4 | 113.3 |
| 115.8 | 110.7 | 114.0 | 110.9 |
| 113.4 | 107.1 | 110.7 | 108.3 |
| 110.3 | 103.0 | 106.3 | 104.7 |
| 107.1 | 78.6 | 105.3 | 101.0 |
| 105.7 | 56.9 | 102.1 | 78.9 |
| 99.8 | 55.4 | 78.4 | 74.4 |
| 78.9 | 54.1 | 55.8 | 56.6 |
| 57.6 | 49.1 | 50.5 | 54.0 |
| 54.3 | 32.8 | 47.2 | 49.5 |
| 49.0 | 29.4 | 29.5 | 46.8 |
| 46.9 | 25.0 | 25.1 | 30.2 |
| 29.4 | 21.7 | 22.7 | 28.4 |
| 26.8 | 17.9 | 18.3 | 26.6 |
| 24.9 | | | 22.8 |
| 21.8 | | | 17.4 |

For the forms A, B and D crystal forms of fumarate, the characteristic peaks (ppm) among the above-described peaks are as follows.
Form A: 124.0, 26.8
Form B: 143.8, 32.8
Form D: 190.5, 138.0

In the present specification, the expression "having a peak at a chemical shift of about 124.0 ppm", for example, means "having a peak essentially equivalent to a chemical shift of 124.0 ppm as measured in a ¹³C solid NMR spectrum under normal measurement conditions or conditions that are essentially the same as those in the present specification".

### Powder X-ray Crystal Diffraction

A sample was ground using an agate mortar, then placed on a X-ray powder diffraction stage and analyzed under the following conditions.

### Measurement Conditions

The measurement conditions are shown in Table 2.

**[Table 2]**

| Sample holder | Glass or copper |
|---|---|
| Target | Copper |
| Detector | Scintillation counter |
| Tube voltage | 40 kV |
| Tube current | 200 mA |
| Slit | DS 1/2°, RS 0.3 mm, SS 1/2° |
| Scan speed | 2°/min |
| Sampling interval | 0.02° |
| Scan range | 5 to 40°C |
| Goniometer | Vertical goniometer |

The X-ray diffraction patterns of the forms A to D crystal forms of fumarate and crystalline of tartrate are shown in Tables 5 to 9, and results of the peak searches are shown in Tables 3 to 7. Further, a list of the characteristic peaks for the respective fumarate crystals is as follows.
Form A 2θ: 18.22, 30.92
Form B 2θ: 27.61, 32.70
Form C 2θ: 9.84, 19.71
Form D 2θ: 8.32, 14.06

Generally, since an error within ±0.2° of the diffraction angle (2θ) can occur in X-ray powder diffraction, the above diffraction angle values need to be understood as including values within a range of about ±0.2° therefrom. Therefore, the present invention includes not only crystals whose peak diffraction angles in X-ray powder diffraction exactly match, but also includes crystals whose peak diffraction angles match within an error of about ±0.2°.

Results of the X-ray powder diffraction peak search of the fumarate form A crystal are shown in Table 3.

**[Table 3-1]**

| 2θ | Half width | d value | Intensity | Relative intensity |
|---|---|---|---|---|
| 7.58 | 0.188 | 11.65 | 1573 | 9 |
| 8.38 | 0.141 | 10.54 | 3395 | 19 |
| 8.62 | 0.165 | 10.25 | 4328 | 25 |
| 9.12 | 0.188 | 9.69 | 2317 | 13 |
| 11.96 | 0.19 | 7.39 | 4332 | 25 |
| 13.18 | 0.21 | 6.71 | 3722 | 21 |
| 14.70 | 0.24 | 6.02 | 3662 | 21 |
| 15.22 | 0.21 | 5.82 | 7117 | 41 |
| 15.70 | 0.21 | 5.64 | 2950 | 17 |
| 16.50 | 0.21 | 5.37 | 3750 | 21 |
| 16.86 | 0.12 | 5.25 | 883 | 5 |
| 17.32 | 0.21 | 5.12 | 3302 | 19 |
| 17.78 | 0.12 | 4.98 | 1302 | 7 |
| 18.22 | 0.33 | 4.87 | 6063 | 35 |
| 18.88 | 0.17 | 4.70 | 1415 | 8 |
| 19.54 | 0.19 | 4.54 | 1978 | 11 |
| 19.86 | 0.19 | 4.47 | 2123 | 12 |
| 20.20 | 0.14 | 4.39 | 1570 | 9 |
| 20.94 | 0.35 | 4.24 | 8297 | 47 |
| 21.46 | 0.21 | 4.14 | 17545 | 100 |
| 21.76 | 0.12 | 4.08 | 2607 | 15 |
| 22.32 | 0.26 | 3.98 | 6130 | 35 |
| 22.72 | 0.21 | 3.91 | 5827 | 33 |
| 22.96 | 0.12 | 3.87 | 2583 | 15 |
| 23.54 | 0.21 | 3.78 | 7315 | 42 |
| 24.34 | 0.19 | 3.65 | 5443 | 31 |
| 25.26 | 0.19 | 3.52 | 3472 | 20 |
| 25.80 | 0.40 | 3.45 | 2963 | 17 |
| 26.72. | 0.19 | 3.33 | 1822 | 10 |
| 26.98 | 0.21 | 3.30 | 3305 | 19 |

**[Table 3-2]**

| 2θ | Half width | d value | Intensity | Relative intensity |
|---|---|---|---|---|
| 27.32 | 0.19 | 3.26 | 1652 | 9 |
| 27.70 | 0.19 | 3.22 | 1412 | 8 |
| 28.22 | 0.19 | 3.16 | 1532 | 9 |
| 28.66 | 0.14 | 3.11 | 1002 | 6 |
| 29.10 | 0.26 | 3.07 | 1542 | 9 |
| 29.32 | 0.12 | 3.04 | 1532 | 9 |
| 29.76 | 0.24 | 3.00 | 1493 | 9 |
| 30.92 | 0.24 | 2.89 | 4577 | 26 |
| 31.28 | 0.19 | 2.86 | 1942 | 11 |
| 31.76 | 0.12 | 2.82 | 762 | 4 |
| 32.36 | 0.17 | 2.76 | 822 | 5 |
| 32.76 | 0.14 | 2.73 | 892 | 5 |
| 33.42 | 0.31 | 2.68 | 1507 | 9 |
| 34.18 | 0.26 | 2.62 | 1482 | 8 |
| 34.68 | 0.14 | 2.58 | 932 | 5 |
| 35.12 | 0.17 | 2.55 | 798 | 5 |
| 35.48 | 0.12 | 2.53 | 803 | 5 |
| 35.88 | 0.12 | 2.50 | 745 | 4 |
| 36.40 | 0.12 | 2.47 | 678 | 4 |
| 37.50 | 0.24 | 2.40 | 983 | 6 |
| 37.94 | 0.17 | 2.37 | 808 | 5 |

Results of the X-ray powder diffraction peak search of the form B crystal form of fumarate are shown in Table 4.

**[Table 4-1]**

| 2θ | Half width | d value | Intensity | Relative intensity |
|---|---|---|---|---|
| 8.71 | 0.22 | 10.14 | 10710 | 42 |
| 10.86 | 0.21 | 8.14 | 2317 | 9 |
| 11.54 | 0.24 | 7.66 | 1280 | 5 |
| 12.58 | 0.11 | 7.03 | 1050 | 4 |
| 13.03 | 0.17 | 6.79 | 8397 | 33 |
| 13.49 | 0.13 | 6.56 | 1620 | 6 |
| 14.19 | 0.17 | 6.24 | 2427 | 9 |
| 14.69 | 0.29 | 6.03 | 6797 | 26 |
| 15.25 | 0.25 | 5.81 | 2180 | 8 |
| 15.84 | 0.20 | 5.59 | 1097 | 4 |
| 16.37 | 0.15 | 5.41 | 4717 | 18 |
| 16.53 | 0.14 | 5.36 | 6477 | 25 |
| 17.00 | 0.12 | 5.21 | 2657 | 10 |
| 17.45 | 0.31 | 5.08 | 15177 | 59 |
| 18.00 | 0.17 | 4.92 | 1333 | 5 |
| 18.52 | 0.24 | 4.79 | 1680 | 7 |
| 19.31 | 0.26 | 4.59 | 3057 | 12 |
| 20.20 | 0.14 | 4.39 | 4300 | 17 |
| 20.53 | 0.21 | 4.32 | 2747 | 11 |
| 20.99 | 0.11 | 4.23 | 1527 | 6 |
| 21.81 | 0.28 | 4.07 | 25673 | 100 |
| 22.24 | 0.17 | 3.99 | 13717 | 53 |
| 22.83 | 0.15 | 3.89 | 10063 | 39 |
| 23.14 | 0.21 | 3.84 | 8827 | 34 |
| 24.17 | 0.20 | 3.68 | 5027 | 20 |
| 24.60 | 0.26 | 3.62 | 6067 | 24 |
| 25.38 | 0.15 | 3.51 | 1503 | 6 |
| 26.32 | 0.22 | 3.38 | 4790 | 19 |
| 27.10 | 0.22 | 3.28 | 6907 | 27 |
| 27.61 | 0.28 | 3.23 | 8233 | 32 |

**[Table 4-2]**

| 2θ | Half width | d value | Intensity | Relative intensity |
|---|---|---|---|---|
| 27.98 | 0.14 | 3.19 | 1607 | 6 |
| 28.37 | 0.11 | 3.14 | 1487 | 6 |
| 28.49 | 0.11 | 3.13 | 1467 | 6 |
| 28.63 | 0.19 | 3.12 | 1487 | 6 |
| 29.32 | 0.31 | 3.04 | 2800 | 11 |
| 29.93 | 0.17 | 2.98 | 3350 | 13 |
| 30.30 | 0.21 | 2.95 | 1563 | 6 |
| 30.62 | 0.31 | 2.90 | 3057 | 12 |
| 31.12 | 0.13 | 2.87 | 1883 | 7 |
| 31.47 | 0.17 | 2.84 | 2243 | 9 |
| 31.95 | 0.14 | 2.80 | 1487 | 6 |
| 32.25 | 0.14 | 2.77 | 1520 | 6 |
| 32.70 | 0.25 | 2.74 | 3977 | 15 |
| 33.02 | 0.15. | 2.71 | 2677 | 10 |
| 33.46 | 0.12 | 2.67 | 1080 | 4 |
| 33.97 | 0.15 | 2.64 | 1927 | 8 |
| 34.58 | 0.18 | 2.59 | 1067 | 4 |
| 34.95 | 0.20 | 2.57 | 2663 | 10 |
| 35.70 | 0.12 | 2.51 | 1350 | 5 |
| 36.83 | 0.20 | 2.44 | 1230 | 5 |
| 36.98 | 0.13 | 2.43 | 1320 | 5 |
| 38.48 | 0.14 | 2.34 | 1433 | 6 |
| 38.84 | 0.22 | 2.32 | 1877 | 7 |
| 39.75 | 0.22 | 2.27 | 1167 | 5 |

Results of the X-ray powder diffraction peak search of the form C crystal form of fumarate are shown in Table 5.

**[Table 5-1]**

| 2θ | Half width | d value | Intensity | Relative intensity |
|---|---|---|---|---|
| 7.62 | 0.24 | 11.59 | 1230 | 7 |
| 7.90 | 0.13 | 11.18 | 1817 | 11 |
| 8.16 | 0.15 | 10.83 | 1223 | 7 |
| 8.47 | 0.15 | 10.43 | 2110 | 12 |
| 8.86 | 0.34 | 9.97 | 4463 | 26 |
| 9.21 | 0.18 | 9.59 | 1190 | 7 |
| 9.84 | 0.18 | 8.98 | 2580 | 15 |
| 10.63 | 0.15 | 8.32 | 1477 | 9 |
| 11.22 | 0.21 | 7.88 | 1003 | 6 |
| 12.05 | 0.17 | 7.34 | 2027 | 12 |
| 12.62 | 0.14 | 7.01 | 2257 | 13 |
| 13.04 | 0.18 | 6.78 | 2313 | 14 |
| 13.43 | 0.14 | 6.59 | 4047 | 24 |
| 13.67 | 0.14 | 6.47 | 2037 | 12 |
| 14.49 | 0.13 | 6.11 | 1863 | 11 |
| 14.77 | 0.12 | 5.99 | 2067 | 12 |
| 15.11 | 0.21 | 5.86 | 8410 | 49 |
| 15.77 | 0.15 | 5.61 | 12537 | 73 |
| 16.08 | 0.12 | 5.51 | 1233 | 7 |
| 16.59 | 0.14 | 5.34 | 2040 | 12 |
| 16.92 | 0.18 | 5.24 | 2663 | 16 |
| 17.53 | 0.17 | 5.05 | 5670 | 33 |
| 17.71 | 0.17 | 5.00 | 8060 | 47 |
| 18.26 | 0.20 | 4.85 | 3080 | 18 |
| 18.99 | 0.18 | 4.67 | 2577 | 15 |
| 19.28 | 0.21 | 4.60 | 3473 | 20 |
| 19.71 | 0.20 | 4.50 | 16880 | 99 |
| 20.43 | 0.19 | 4.34 | 6840 | 40 |
| 20.96 | 0.21 | 4.23 | 5483 | 32 |
| 21.50 | 0.22 | 4.13 | 14233 | 83 |

**[Table 5-2]**

| 2θ | Half width | d value | Intensity | Relative intensity |
|---|---|---|---|---|
| 21.87 | 0.14 | 4.06 | 8810 | 52 |
| 22.07 | 0.15 | 4.02 | 8143 | 48 |
| 22.66 | 0.19 | 3.92 | 17063 | 100 |
| 23.07 | 0.26 | 3.85 | 6393 | 37 |
| 23.60 | 0.25 | 3.77 | 7057 | 41 |
| 23.92 | 0.18 | 3.72 | 4160 | 24 |
| 24.43 | 0.21 | 3.64 | 2853 | 17 |
| 24.88 | 0.24 | 3.58 | 5030 | 29 |
| 25.35 | 0.31 | 3.51 | 4497 | 26 |
| 25.98 | 0.20 | 3.43 | 2057 | 12 |
| 26.62 | 0.18 | 3.35 | 7817 | 46 |
| 27.04 | 0.15 | 3.29 | 2947 | 17 |
| 27.28 | 0.20 | 3.27 | 3540 | 21 |
| 27.88 | 0.12 | 3.20 | 3307 | 19 |
| 27.98 | 0.14 | 3.19 | 4030 | 24 |
| 28.27 | 0.17 | 3.15 | 1603 | 9 |
| 28.74 | 2.00 | 3.10 | 1997 | 12 |
| 28.97 | 0.12 | 3.08 | 1997 | 12 |
| 29.18 | 0.13 | 3.06 | 1617 | 9 |
| 29.63 | 0.20 | 3.01 | 3380 | 20 |
| 30.23 | 0.19 | 2.95 | 1937 | 11 |
| 30.63 | 0.15 | 2.92 | 1630 | 10 |
| 30.96 | 0.22 | 2.89 | 2460 | 14 |
| 31.31 | 0.17 | 2.85 | 2253 | 13 |
| 31.67 | 0.24 | 2.82 | 4503 | 26 |
| 32.27 | 0.12 | 2.77 | 1387 | 8 |
| 32.67 | 0.21 | 2.74 | 1327 | 8 |
| 33.44 | 0.20 | 2.68 | 1750 | 10 |
| 34.13 | 0.21 | 2.62 | 2170 | 13 |
| 34.45 | 0.14 | 2.60 | 1250 | 7 |

**[Table 5-3]**

| 2θ | Half width | d value | Intensity | Relative intensity |
|---|---|---|---|---|
| 34.75 | 0.11 | 2.58 | 1070 | 6 |
| 35.25 | 0.25 | 2.54 | 2350 | 14 |
| 35.84 | 0.21 | 2.50 | 1343 | 8 |
| 36.78 | 0.14 | 2.44 | 1453 | 9 |
| 37.57 | 0.12 | 2.39 | 1357 | 8 |
| 37.95 | 0.17 | 2.37 | 1120 | 7 |
| 38.40 | 0.13 | 2.34 | 1540 | 9 |
| 38.73 | 0.12 | 2.32 | 1360 | 8 |

Results of the X-ray powder diffraction peak search of the form D crystal form of fumarate are shown in Table 6.

**[Table 6-1]**

| **2θ** | Half width | d value | Intensity | Relative intensity |
|---|---|---|---|---|
| 8.32 | 0.18 | 10.62 | 6100 | 45 |
| 10.84 | 0.12 | 8.15 | 2357 | 17 |
| 11.00 | 0.18 | 8.04 | 2963 | 22 |
| 11.54 | 0.25 | 7.66 | 8513 | 63 |
| 14.06 | 0.20 | 6.29 | 8780 | 65 |
| 15.31 | 0.21 | 5.78 | 1627 | 12 |
| 15.68 | 0.18 | 5.65 | 2640 | 19 |
| 15.82 | 0.14 | 5.60 | 2457 | 18 |
| 16.56 | 0.21 | 5.35 | 9440 | 70 |
| 17.08 | 0.12 | 5.19 | 2900 | 21 |
| 17.18 | 0.12 | 5.16 | 3133 | 23 |
| 17.58 | 0.24 | 5.04 | 3717 | 27 |
| 17.95 | 0.19 | 4.94 | 4690 | 35 |
| 18.40 | 0.21 | 4.82 | 4107 | 30 |
| 18.58 | 0.12 | 4.77 | 3553 | 26 |
| 19.67 | 0.13 | 4.51 | 2120 | 16 |
| 20.28 | 0.32 | 4.38 | 3500 | 26 |
| 20.95 | 0.27 | 4.24 | 13553 | 100 |
| 21.53 | 0.11 | 4.12 | 3253 | 24 |
| 21.61 | 0.11 | 4.11 | 3373 | 25 |
| 21.99 | 0.11 | 4.04 | 4327 | 32 |
| 22.12 | 0.11 | 4.02 | 4237 | 31 |
| 22.45 | 0.21 | 3.96 | 5097 | 38 |
| 22.83 | 0.24 | 3.89 | 5137 | 18 |
| 23.49 | 0.18 | 3.78 | 2420 | 18 |
| 23.90 | 0.22 | 3.72 | 2413 | 25 |
| 24.53 | 0.11 | 3.63 | 3340 | 30 |
| 24.67 | 0.17 | 3.61 | 4013 | 19 |
| 25.04 | 0.20 | 3.55 | 2603 | 19 |
| 25.57 | 0.15 | 3.48 | 2617 | 20 |

**[Table 6-2]**

| 2θ | Half width | d value | Intensity | Relative intensity |
|---|---|---|---|---|
| 25.81 | 0.12 | 3.45 | 2773 | 20 |
| 26.06 | 0.22 | 3.42 | 2737 | 25 |
| 26.56 | 0.26 | 3.35 | 3430 | 22 |
| 27.43 | 0.27 | 3.25 | 2980 | 22 |
| 27.79 | 0.21 | 3.21 | 2963 | 15 |
| 28.49 | 0.11 | 3.13 | 2013 | 15 |
| 28.58 | 0.11 | 3.12 | 1983 | 32 |
| 29.16 | 0.33 | 3.06 | 4307 | 11 |
| 30.03 | 0.14 | 2.97 | 1470 | 12 |
| 30.15 | 0.11 | 2.96 | 1593 | 9 |
| 30.72 | 0.11 | 2.91 | 1243 | 11 |
| 31.12 | 0.12 | 2.87 | 1450 | 9 |
| 31.79 | 0.12 | 2.81 | 1233 | 10 |
| 32.62 | 0.12 | 2.74 | 1297 | 11 |
| 33.10 | 0.20 | 2.70 | 1460 | 10 |
| 33.38 | 0.15 | 2.68 | 1317 | 12 |
| 35.08 | 0.13 | 2.56 | 1663 | 11 |
| 37.50 | 0.18 | 2.40 | 1473 | 11 |

Results of the X-ray powder diffraction peak search of the crystalline of tartrate are shown in Table 7.

**[Table 7-1]**

| **2θ** | Half width | d value | Intensity | Relative intensity |
|---|---|---|---|---|
| 6.64 | 0.14 | 13.30 | 3421 | 25 |
| 7.56 | 0.24 | 11.68 | 13771 | 100 |
| 10.06 | 0.17 | 8.79 | 2712 | 20 |
| 10.46 | 0.14 | 8.45 | 1912 | 14 |
| 10.88 | 0.21 | 8.13 | 5012 | 36 |
| 12.58 | 0.21 | 7.03 | 7758 | 56 |
| 13.94 | 0.14 | 6.35 | 1238 | 9 |
| 15.18 | 0.38 | 5.83 | 4438 | 32 |
| 15.68 | 0.21 | 5.65 | 4200 | 30 |
| 15.94 | 0.21 | 5.56 | 4883 | 35 |
| 16.82 | 0.17 | 5.27 | 4329 | 31 |
| 17.42 | 0.21 | 5.09 | 5279 | 38 |
| 18.12 | 0.38 | 4.89 | 2654 | 19 |
| 19.00 | 0.17 | 4.67 | 4888 | 35 |
| 19.28 | 0.12 | 4.60 | 2046 | 15 |
| 19.70 | 0.14 | 4.50 | 2350 | 17 |
| 20.18 | 0.24 | 4.40 | 2342 | 17 |
| 21.00 | 0.24 | 4.23 | 13738 | 100 |
| 21.84 | 0.40 | 4.07 | 7950 | 58 |
| 22.36 | 0.26 | 3.97 | 9412 | 68 |
| 22.86 | 0.12 | 3.89 | 3300 | 24 |
| 23.84 | 0.14 | 3.73 | 2612 | 19 |
| 24.30 | 0.19 | 3.66 | 2879 | 21 |
| 24.84 | 0.17 | 3.58 | 4104 | 30 |
| 25.40 | 0.33 | 3.50 | 3496 | 25 |
| 26.00 | 0.19 | 3.42 | 2308 | 17 |
| 26.62 | 0.19 | 3.35 | 2208 | 16. |
| 27.66 | 0.24 | 3.22 | 7250 | 53 |
| 28.66 | 0.31 | 3.11 | 2625 | 19 |
| 29.18 | 0.12 | 3.06 | 1700 | 12 |

**[Table 7-2]**

| 2θ | Half width | d value | Intensity | Relative intensity |
|---|---|---|---|---|
| 29.36 | 0.17 | 3.04 | 1521 | 11 |
| 29.76 | 0.24 | 3.00 | 1596 | 12 |
| 30.10 | 0.14 | 2.97 | 1312 | 10 |
| 30.58 | 0.17 | 2.92 | 1554 | 11 |
| 32.20 | 0.14 | 2.78 | 1521 | 11 |
| 32.70 | 0.21 | 2.74 | 1950 | 14 |
| 33.10 | 0.12 | 2.70 | 1296 | 9 |
| 33.56 | 0.19 | 2.67 | 1771 | 13 |
| 33.66 | 0.14 | 2.66 | 1625 | 12 |
| 34.16 | 0.14 | 2.62 | 1238 | 9 |
| 34.36 | 0.12 | 2.61 | 1325 | 10 |
| 35.28 | 0.12 | 2.54 | 1604 | 12 |
| 35.42 | 0.17 | 2.53 | 1592 | 12 |
| 37.16 | 0.12 | 2.42 | 1138 | 8 |

### Infrared Spectrophotometry

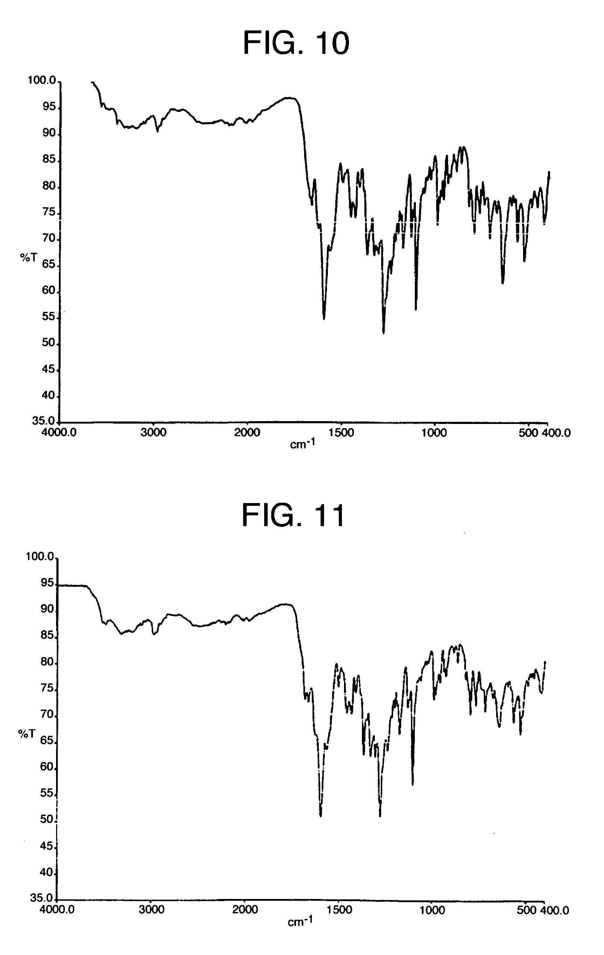

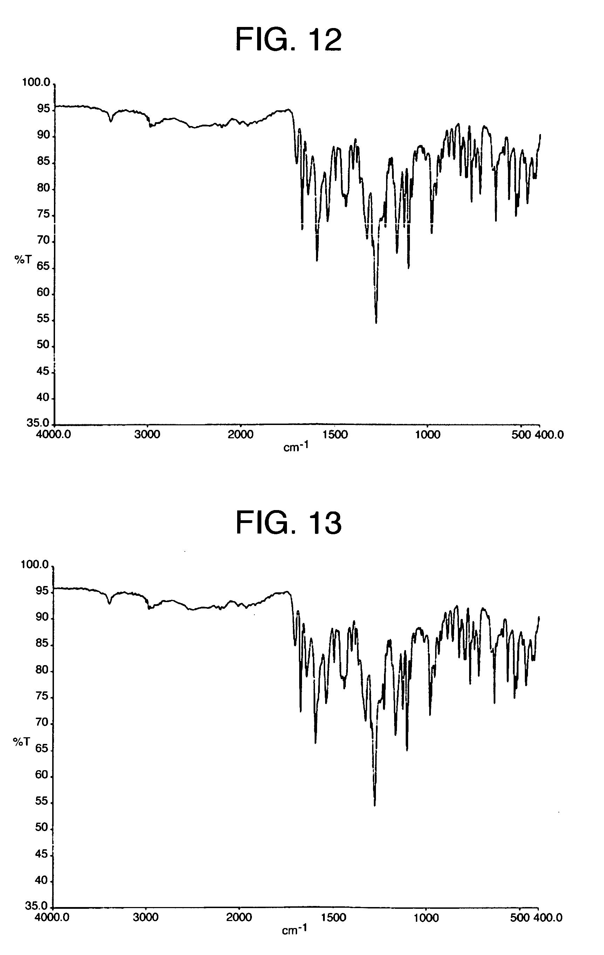

Infrared spectrophotometry of the crystals obtained in the respective working examples was carried out using an FT-IR Spectrum-One manufactured by PerkinElmer Japan Co., Ltd., at a measurement range of 4,000 to 400 cm⁻¹ and resolution of 4 cm⁻¹ according to the ATR method of Infrared spectrophotometry described in the Japanese Pharmacopoeia Fourteenth Edition, General Test Methods.
The infrared spectra of the forms A to D crystal forms of fumarates and crystal of tartrate are shown in Figs. 10 to 14, and the respective crystal spectrum peaks are shown in Tables 8 to 12.

The infrared spectra peak for the fumarate form A crystal is shown in Table 8.

**[Table 8]**

| Peak No. | Wavenumber (cm⁻¹) | Peak No. | wavenumber (cm⁻¹) | Peak No. | Wavenumber (cm⁻¹) |
|---|---|---|---|---|---|
| 1 | 3197 | 17 | 1215 | 33 | 823 |
| 2 | 2968 | 18 | 1201 | 34 | 792 |
| 3 | 2208 | 19 | 1188 | 35 | 766 |
| 4 | 2029 | 20 | 1174 | 36 | 756 |
| 5 | . 1664 | 21 | 1130 | 37 | 741 |
| 6 | 1596 | 22 | 1119 | 38 | 711 |
| 7 | 1566 | 23 | 1104 | 39 | 676 |
| 8 | 1499 | 24 | 1061 | 40 | 643 |
| 9 | 1456 | 25 | 1027 | 41 | 597 |
| 10 | 1433 | 26 | 991 | 42 | 566 |
| 11 | 1411 | 27 | 981 | 43 | 531 |
| 12 | 1368 | 28 | 971 | 44 | 491 |
| 13 | 1332 | 29 | 958 | 45 | 460 |
| 14 | 1308 | 30 | 936 | 46 | 426 |
| 15 | 1277 | 31 | 890 | | |
| 16 | 1239 | 32 | 863 | | |

The infrared spectra peak for the form B crystal form of fumarate is shown in Table 9.

**[Table 9]**

| Peak No. | Wavenumber (cm⁻¹) | Peak No. | Wavenumber (cm⁻¹) | Peak No. | Wavenumber (cm⁻¹) |
|---|---|---|---|---|---|
| 1 | 3320 | 17 | 1240 | 33 | 643 |
| 2 | 2969 | 18 | 1201 | 34 | 565 |
| 3 | 2485 | 19 | 1173 | 35 | 531 |
| 4 | 1980 | 20 | 1129 | 36 | 492 |
| 5 | 1682 | 21 | 1103 | 37 | 458 |
| 6 | 1663 | 22 | 991 | 38 | 419 |
| 7 | 1596 | 23 | 981 | | |
| 8 | 1564 | 24 | 958 | | |
| 9 | 1504 | 25 | 926 | | |
| 10 | 1458 | 26 | 884 | | |
| 11 | 1432 | 27 | 863 | | |
| 12 | 1412 | 28 | 821 | | |
| 13 | 1368 | 29 | 794 | | |
| 14 | 1333 | 30 | 766 | | |
| 15 | 1306 | 31 | 717 | | |
| 16 | 1278 | 32 | 674 | | |

The infrared spectra peak for the form C crystal form of fumarate is shown in Table 10.

**[Table 10]**

| Peak No. | Wavenumber (cm⁻¹) | Peak No. | Wavenumber (cm⁻¹) | Peak No. | Wavenumber (cm⁻¹) |
|---|---|---|---|---|---|
| 1 | 3198 | 17 | 1202 | 33 | 743 |
| 2 | 2967 | 18 | 1174 | 34 | 714 |
| 3 | 2205 | 19 | 1129 | 35 | 677 |
| 4 | 1675 | 20 | 1120 | 36 | 640 |
| 5 | 1634 | 21 | 1103 | 37 | 595 |
| 6 | 1597 | 22 | 1028 | 38 | 568 |
| 7 | 1499 | 23 | 991 | 39 | 531 |
| 8 | 1457 | 24 | 969 | 40 | 459 |
| 9 | 1433 | 25 | 959 | 41 | 425 |
| 10 | 1409 | 26 | 936 | | |
| 11 | 1366 | 27 | 897 | | |
| 12 | 1323 | 28 | 863 | | |
| 13 | 1307 | 29 | 803 | | |
| 14 | 1277 | 30 | 793 | | |
| 15 | 1232 | 31 | 765 | | |
| 16 | 1215 | 32 | 756 | | |

The infrared spectra peak for the form D crystal form of fumarate is shown in Table 11.

**[Table 11]**

| Peak No. | Wavenumber (cm⁻¹) | Peak No. | Wavenumber (cm⁻¹) | Peak No. | Wavenumber (cm⁻¹) |
|---|---|---|---|---|---|
| 1 | 3397 | 17 | 1280 | 33 | 828 |
| 2 | 2970 | 18 | 1231 | 34 | 800 |
| 3 | 2209 | 19 | 1202 | 35 | 792 |
| 4 | 1966 | 20 | 1169 | 36 | 769 |
| 5 | 1708 | 21 | 1128 | 37 | 745 |
| 6 | 1678 | 22 | 1105 | 38 | 721 |
| 7 | 1647 | 23 | 1088 | 39 | 637 |
| 8 | 1599 | 24 | 1064 | 40 | 593 |
| 9 | 1542 | 25 | 1029 | 41 | 567 |
| 10 | 1499 | 26 | 1013 | 42 | 529 |
| 11 | 1444 | 27 | 982 | 43 | 519 |
| 12 | 1407 | 28 | 959 | 44 | 489 |
| 13 | 1386 | 29 | 937 | 45 | 470 |
| 14 | 1370 | 30 | 924 | 46 | 436 |
| 15 | 1331 | 31 | 890 | 47 | 426 |
| 16 | 1302 | 32 | 862 | | |

The infrared spectra peak for the crystal of tartrate is shown in Table 12.

**[Table 12]**

| Peak No. | Wavenumber (cm⁻¹) | Peak No. | Wavenumber (cm⁻¹) |
|---|---|---|---|
| 1 | 3402 | 18 | 1129 |
| 2 | 2973 | 19 | 1105 |
| 3 | 2164 | 20 | 1067 |
| 4 | 1660 | 21 | 960 |
| 5 | 1599 | 22 | 925 |
| 6 | 1562 | 23 | 886 |
| 7 | 1500 | 24 | 836 |
| 8 | 1458 | 25 | 818 |
| 9 | 1443 | 26 | 792 |
| 10 | 1408 | 27 | 772 |
| 11 | 1372 | 28 | 727 |
| 12 | 1331 | 29 | 601 |
| 13 | 1305 | 30 | 567 |
| 14 | 1280 | 31 | 529 |
| 15 | 1233 | 32 | 426 |
| 16 | 1203 | 33 | 407 |
| 17 | 1174 | | |

### Thermal Analysis Measurement

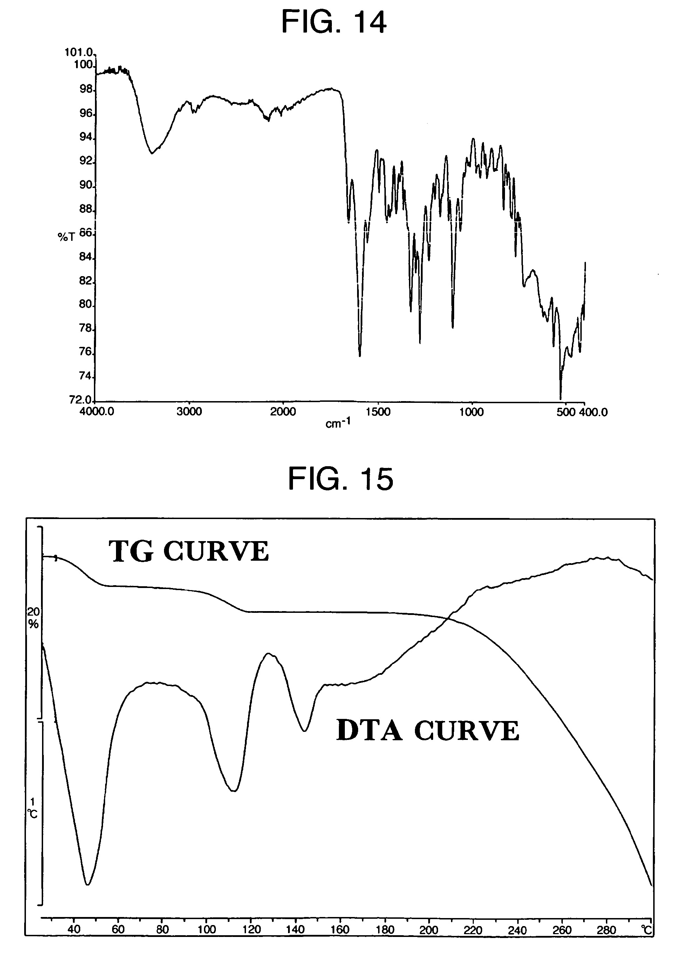

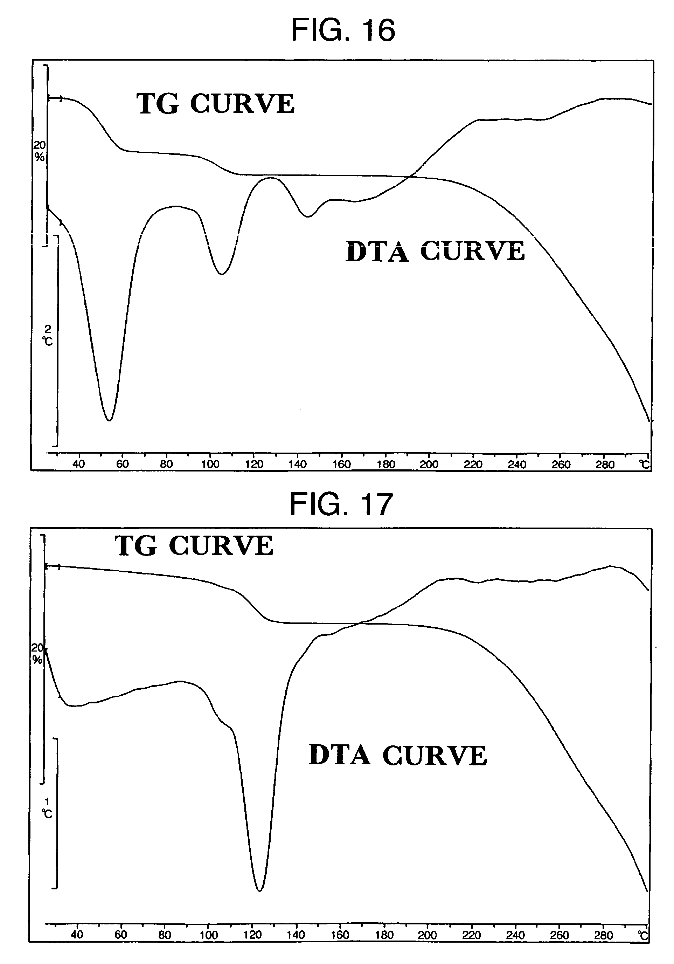

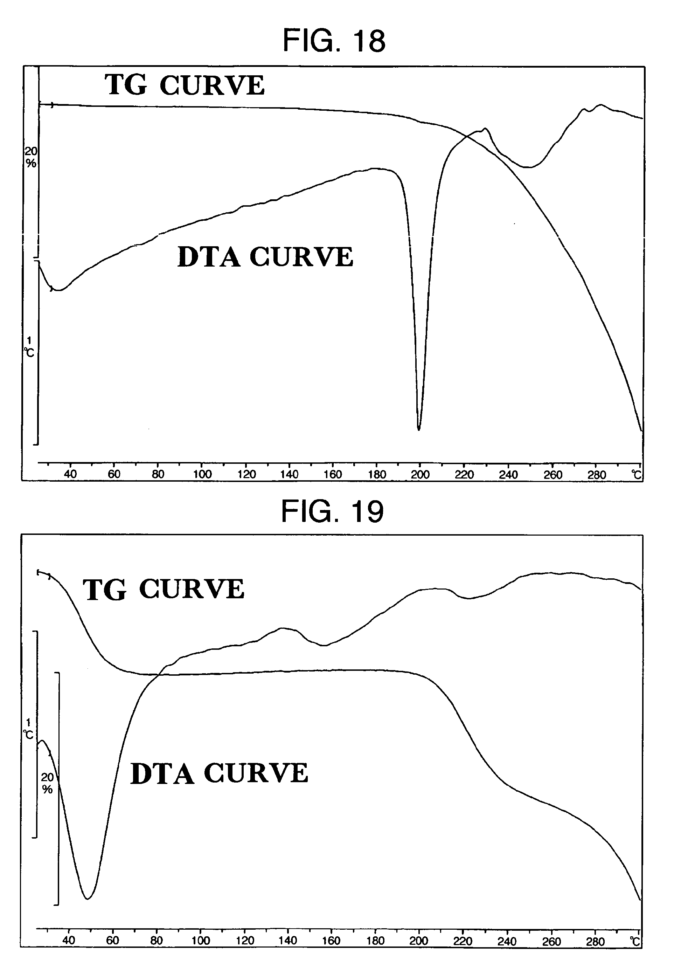

The thermal analysis measurement of the crystals obtained in the respective working examples was carried out under a nitrogen gas flow with a rate of temperature increase of 10°C/min in a measurement range of 25 to 300°C using the thermal analysis system TGA/SDTA851^{e} manufactured by Mettler-Toledo K.K. with an A1 sample pan.
The thermal analysis results of the crystals (TG-DTA curve) are shown in Figs. 15 to 19. Further, a list of the characteristic endothermic peaks for the respective fumarate crystals is as follows.
Form A: 46°C, 112°C, 143°C
Form B: 54°C, 105°C, 143°C
Form C: 121°C
Form D: 200°C

### General Production Process

The process for producing a crystal of the compound (i) represented by general formula (i) according to the present invention is illustrated below.

The crystal according to the present invention can be produced stably on an industrial scale by producing the compound (i) according to the processes illustrated in the following production examples, heating the compound (i) and fumaric acid or tartaric acid in a specific solvent for dissolution and then cooling the resultant solution while stirring for crystallization, or recrystallization of a fumarate or tartrate of the obtained compound (i).

The compound (i) used in the crystallization may be in any form, including as a hydrate, an anhydride, amorphous, crystal (including substances which consist of a plurality of crystal forms), or may even be a mixture of these.

Examples of the solvent used for the crystallization include a single solvent or a mixed solvent containing two or more solvents selected from the group consisting of alcohol solvents such as methanol, ethanol, 2-propanol and *n*-propanol, amide solvents such as acetonitrile and *N,N-*dimethylformamide, ester solvents such as ethyl acetate and water.

The solvent for obtaining a fumarate form A crystal is preferably a mixed solvent of acetone and water. More preferably, the solvent is a mixed solvent of acetone and water having a mixing ratio between 5:1 and 1:5, and most preferably, a mixed solvent of acetone and water having a mixing ratio of 1:3.

The solvent for obtaining a form B crystal form of fumarate is preferably a mixed solvent of n-propanol and water. More preferably, the solvent is a mixed solvent of *n*-propanol and water having a mixing ratio between 5:1 and 1:5, and most preferably, a mixed solvent of *n*-propanol and water having a mixing ratio of 1:3.

The solvent for obtaining a form C crystal form of fumarate is preferably a mixed solvent of methanol and water. More preferably, the solvent is a mixed solvent of methanol and water having a mixing ratio between 5:1 and 1:5, and most preferably, a mixed solvent of methanol and water having a mixing ratio of 3:5.

Although the solvent for obtaining a form D crystal form of fumarate is an alcohol solvent, an amide solvent, an ester solvent or a mixed solvent thereof, an alcohol solvent is preferred. More preferably, the solvent is ethanol or a mixed solvent of ethanol and 2-propanol. Still more preferably, the solvent is a mixed solvent of ethanol and 2-propanol, and most preferably, a mixed solvent of ethanol and 2-propanol having a mixing ratio of 2:3.

The solvent for obtaining a crystal of tartrate is, preferably, a mixed solvent of methanol and water. More preferably, the solvent is a mixed solvent of methanol and water having a mixing ratio between 5:1 and 1:5, and most preferably, a mixed solvent of methanol and water having a mixing ratio of 4:1.

The used amount of the solvent can be appropriately selected with a lower limit set at the amount where compound (i) dissolves by heating and an upper limit set at the amount where the crystal yield amount does not substantially decrease.

The crystals obtained by the above-described process consist of a single crystal form which is stable and is not easily transformed into other crystal forms or into an amorphous substance. Further, these crystals have good physical properties such as not being hygroscopic, and are suitable for drug formulation.

While the temperature for heating compound (i) to dissolve may be appropriately selected depending on the solvent so that compound (i) dissolves, the temperature is preferably from the reflux temperature of the recrystallization solvent to 50°C, and more preferably the temperature is from 65 to 55°C.
If the cooling is carried out rapidly, crystals having different forms, or specifically a product containing multiple forms, are obtained. Therefore, the cooling during crystallization is preferably carried out by appropriately adjusting the cooling temperature in consideration of the effects on quality, grain size or the like of the crystals. Slow cooling is preferred, specifically, cooling at a rate of from 30 to 5°C per hour, for example, is preferable. A more preferred cooling temperature is from 30 to 20°C per hour.
Further, while the final crystallization temperature may be appropriately selected according to the crystal yield amount, quality and the like, from room temperature to 60°C is preferred.
The crystallized crystals are separated by a normal filtering operation, optionally washed with a solvent and then dried to obtain the desired crystals. Many of the solvents used in the washing of the crystals are the same as the crystallization solvents.

### Crystal Drying Method

Crystals separated by the filtering operation can be dried by, as appropriate, leaving out in air or under a nitrogen gas flow, or by heating.
Regarding the drying time, the time until residual solvent falls below a certain level can be appropriately selected according to the production amount, the drying apparatus, the drying temperature and the like. The drying may also be carried out under ventilation or under reduced pressure. The degree of reduced pressure may be appropriately selected according to the production amount, the drying apparatus, the drying temperature and the like. After the drying, the obtained crystals can optionally be left in air.

After dissolving the crystals of 1-{1-[2-(7-methoxy-2,2-dimethyl-4-oxochroman-8-yl)-ethyl]piperidin-4-yl}-*N*-methyl-1*H*-indole-6-carboxamide fumarate crystal or crystal of tartrate according to the present invention in a solvent, an amorphous product of the compound can be obtained by a known method such as freeze-drying.

The crystal form of fumarate or of tartrate of compound (i) according to the present invention (hereinafter sometimes simply referred to as "crystal") exhibits an excellent effects and efficacy as a drug, and is effective in preventing or treating lower urinary tract symptoms, cognitive impairment associated with Alzheimer's disease or senile dementia, learning or memory disorder, or anxiety disorder, schizophrenia, emotional disorder, alcohol and/or cocaine dependence, nicotine addiction or symptoms associated with smoking cessation, or visual attention disorder and the like. The fumarate crystal or crystalline of tartrate of compound (i) according to the present invention is especially effective in preventing or treating lower urinary tract symptoms such as urinary storage symptoms, urinary frequency or urinary incontinence.

The preventive or therapeutic agent according to the present invention can be formulated by common methods. Preferred dosage forms include as a tablet, a powder, a fine granule, a granule, a coated tablet, a capsule, a syrup, a troche, an inhalant, a suppository, an injection, an ointment, an eye drop, an eye ointment, a nasal drop, an ear drop, a poultice and a lotion. For formulation, commonly used additives may be used. Examples of such an additive include an excipient, a binder, a lubricant, a coloring agent, a flavoring agent, as well as, a stabilizer, an emulsifier, an adsorption enhancer, a surfactant, a pH regulator, an antiseptic and an antioxidant, as necessary. These agents can be formulated by blending ingredients that are commonly used as raw materials for pharmaceutical formulations according to common methods.

Examples of such ingredients include animal or vegetable oils such as soybean oil, tallow or synthetic glyceride; hydrocarbons such as liquid paraffin, squalane or solid paraffin; ester oils such as octyldodecyl myristate or isopropyl myristate; higher alcohols such as cetostearyl alcohol or behenyl alcohol; silicone resins; silicone oils, surfactants such as polyoxyethylene fatty acid esters, sorbitan fatty acid esters, glycerin fatty acid esters, polyoxyethylene sorbitan fatty acid esters, polyoxyethylene hydrogenated castor oil or a polyoxyethylene-polyoxypropylene block copolymer; water-soluble polymers such as hydroxyethyl cellulose, polyacrylic acid, a carboxyvinyl polymer, polyethylene glycol, polyvinylpyrrolidone or methyl cellulose; lower alcohols such as ethanol or isopropanol; polyvalent alcohols such as glycerin, propylene glycol, dipropylene glycol or sorbitol; sugars such as glucose or sucrose; inorganic powders such as silicic acid anhydride, magnesium aluminum silicate or aluminum silicate; and purified water. Examples of an excipient include lactose, corn starch, saccharose, glucose, mannitol, sorbit, crystalline cellulose and silicon dioxide. Examples of a binder include polyvinyl alcohol, polyvinyl ether, methylcellulose, ethylcellulose, gum Arabic, Tragacanth, gelatin, shellac, hydroxypropylmethylcellulose, hydroxypropylcellulose, polyvinylpyrrolidone, a polypropylene glycol-polyoxyethylene block polymer and meglumine. Examples of the disintegrant include starch, agar, gelatin powder, crystalline cellulose, calcium carbonate, sodium bicarbonate, calcium citrate, dextrin, pectin, and carboxymethylcellulose calcium. Examples of the lubricant include magnesium stearate, talc, polyethylene glycol, silica, and hydrogenated vegetable oil. Examples of the coloring agent include products which are allowed for addition to pharmaceuticals. Examples of a flavoring agent include cocoa powder, menthol, aromatic powder, peppermint oil, borneol and cinnamon powder.

In the case of an oral formulation, for example, the active ingredient crystals and an excipient, and optionally a binder, a disintegrant, a lubricant, a coloring agent, a flavoring agent and the like are added, and then the resultant mixture is formulated into, for example, a powder, a pavule, a granule, a tablet, a coated tablet, a capsule and the like according to a common method. In the case of a tablet or granule, these formulations may obviously be appropriately coated with sugar or some other material as necessary. In the case of a syrup or a formulation used for injection, a pH regulator, a solubilizer or an isotonizing agent, for example, are added, and as necessary, a solubilizing aid, a stabilizer and the like may also be added, and then the resultant mixture is formulated by a common method. In the case of an external preparation, the production method is not limited, and thus can be produced by a common method. Various materials that are commonly used for pharmaceuticals, quasi drugs, cosmetics or the like can be used herein as the base material. Examples of such a material may include animal and vegetable oils, mineral oils, ester oils, waxes, higher alcohols, fatty acids, silicone oils, surfactants, phospholipids, alcohols, polyvalent alcohols, water-soluble polymers, clay minerals and purified water. In addition, a pH regulator, an antioxidant, a chelating agent, antiseptic and antifungal agents, a coloring agent, a perfume or the like may also optionally be added. Moreover, ingredients having differentiation-inducing action, such as a blood flow-promoting agent, an antibacterial agent, an antiphlogistic, a cell activator, vitamins, amino acids, a moisturizer or keratolytic drug may also be optionally blended.

The dosage of the preventive or therapeutic agent according to the present invention is different depending on the degree of symptoms, age, sex, body weight, dosage form, the type of salt, specific type of disease and the like. For an adult, in general, the agent is administered orally, at a dosage approximately between 30 µg and 10 g, preferably between 100 µg and 5 g and more preferably between 100 µg and 100 mg and administered by injection, at a dosage approximately between 30 µg and 1 g, preferably between 100 µg and 500 mg, and more preferably between 100 µg and 30 mg as crystals of fumarate or tartrate of compound (i) of the present invention once or divided into several times per day.

The present invention will now be described in more detail and specifically by the following production examples, working examples, reference examples, test examples and formulation examples. However, the present invention is not intended to be limited by these production examples, working examples, reference examples and formulation examples.

### Production Example 1

### Synthesis of methyl 1-(1-benzyloxycarbonylpiperidin-4-yl)-1H-indole-6-carboxylate

44.3 g of methyl 3-amino-4-(2,2-dimethoxyethyl)benzoate synthesized according to the publication (Tetrahedron Letters, Vol. 37, No. 34, pp. 6045-6048) and 64.9 g of benzyl 4-oxo-1-piperidinecarboxylate were dissolved in 485 mL of acetic acid, and the resultant reaction solution was then stirred at room temperature. Approximately 20 minutes later, 58.9 g of sodium triacetoxyborohydride was added into the reaction solution. The reaction solution was stirred for a further 2 hours and then 485 mL of water was added thereto. The reaction solution was then heated to between 100 and 115°C. Approximately 3 hours later, the reaction solution was cooled, and then concentrated under reduced pressure. Water and ethyl acetate were added thereto so as to separate an organic layer. The obtained organic layer was washed with saturated aqueous sodium bicarbonate and brine, and dried over anhydrous magnesium sulfate. After removing the drying agent by filtration, the organic layer was concentrated under reduced pressure, and the resulting residue was then purified by NH silica gel column chromatography (hexane/ethyl acetate). The obtained solid was suspended in a mixed solvent of hexane and *t*-butylmethyl ether and was then collected by filtration to obtain 64.6 g of the title compound.
¹H-NMR (400 MHz, CDCl₃) δ (ppm): 1.80-2.05 (m, 2H), 2.05-2.23 (m, 2H), 2.92-3.15 (m, 2H), 3.96 (s, 3H), 4.30-4.60 (m, 3H), 5.18 (s, 2H), 6.58 (dd, J=0.4, 2.8 Hz, 1H), 7.30-7.45 (m, 6H), 7.64 (dd, J=0.4, 8.4 Hz, 1H), 7.80 (dd, J=1.6, 8.4 Hz, 1H), 8.14 (s, 1H).

### Production Example 2

### Synthesis of 1-(1-benzyloxycarbonylpiperidin-4-yl)-1H-indole-6-carboxylic acid

90.0 g of methyl 1-(1-benzyloxycarbonylpiperidin-4-yl)-1H-indole-6-carboxylate was dissolved in a mixed solution consisting of 760 mL of methanol and 200 mL of tetrahydrofuran. To the reaction solution was then added 92 mL of 5 N aqueous sodium hydroxide and the reaction mixture was heated to between 60 and 70°C. After the completion of the reaction, the reaction solution was cooled, added 65.0 g of ammonium chloride, and then concentrated under reduced pressure. 5% aqueous potassium sulfate was added to the resulting residueto adjust the pH of the mixture to 5 to 6, and then the mixture was extracted with ethyl acetate. The organic layer was washed with water and brine, and then dried over anhydrous magnesium sulfate. After removing the drying agent by filtration, the organic layer was concentrated under reduced pressure. The resulting residue was solidified from a mixed solvent of hexane and *t*-butylmethyl ether and then collected by filtration to obtain 75.6 g of the title compound.
¹H-NMR (400 MHz, CDCl₃) δ (ppm): 1.80-2.04 (m, 2H), 2.06-2.21 (m, 2H), 2.94-3.16 (m, 2H), 4.30-4.58 (m, 3H), 5.19 (s, 2H), 6.60 (dd, J=0.8, 3.6 Hz, 1H), 7.30-7.44 (m, 6H), 7.68 (dd, J=0.8, 8.4 Hz, 1H), 7.88 (dd, J=1.6, 8.4 Hz, 1H), 8.22 (s, 1H).

### Production Example 3

### Synthesis of N-methyl-1-(1-benzyloxycarbonylpiperidin-4-yl)-1H-indole-6-carboxamide

2.00 g of 1-(1-benzyloxycarbonylpiperidin-4-yl)-1H-indole-6-carboxylic acid was dissolved in 20 mL of tetrahydrofuran, and 1.03 g of 1,1'-carbonylbis-1*H-*imidazole was added the reaction solution. The reaction solution was stirred at room temperature for 1.5 hours, and then 4.11 mL of 40% aqueous methylamine was added thereto. After the completion of the reaction, the reaction solution was extracted with ethyl acetate. The organic layer was washed with saturated aqueous sodium bicarbonate, saturated aqueous ammonium chloride and brine. The organic layer was then dried over anhydrous magnesium sulfate. After removing the drying agent by filtration, the organic layer was concentrated under reduced pressure, and the resulting residue was then purified by NH silica gel column chromatography (ethyl acetate) and silica gel column chromatography (hexane/ethyl acetate) to obtain 1.77 g of the title compound.
¹H-NMR (400 MHz, CDCl₃) δ (ppm): 1.80-2.00 (m, 2H), 2.03-2.17 (m, 2H), 2.90-3.10 (m, 2H), 3.06 (d, J=4.8 Hz, 3H), 4.30-4.58 (m, 3H), 5.16 (s, 2H), 6.21 (brs, 1H), 6.55 (dd, J=0.8, 3.2 Hz, 1H), 7.27 (d, J=3.6 Hz, 1H), 7.28-7.40 (m, 6H), 7.61 (dd, J=0.8, 8.0 Hz, 1H), 8.03 (s, 1H).

### Production Example 4

### Synthesis of N-methyl-1-(piperidin-4-yl)-1H-indole-6-carboxamide

1.77 g of *N*-methyl-1-(1-benzyloxycarbonylpiperidin-4-yl)-1*H*-indole-6 carboxamide was dissolved in 30 mL of methanol, and 200 mg of 10% palladium-carbon was added to the solution. The reaction system was purged with hydrogen, and then the reaction solution was stirred at room temperature. After the completion of the reaction, the 10% palladium-carbon was removed from the reaction solution by filtration, and the reaction solution was then concentrated under reduced pressure. The resulting residue was purified by NH silica gel column chromatography (ethyl acetate/methanol), then solidified from a mixed solution consisting of ethyl acetate, *t*-butylmethyl ether and methanol to obtain 973 mg of the title compound.
¹H-NMR (400 MHz, CDCl₃) δ (ppm): 1.86-1.99 (m, 2H), 2.06-2.14 (m, 2H), 2.84 (dt, J=2.4, 12.4 Hz, 2H), 3.06 (d, J=4.8 Hz, 3H), 3.22-3.30 (m, 2H), 4.44 (tt, J=4.0, 12.0 Hz, 1H), 6.24 (brs, 1H), 6.54 (dd, J=0.8, 3.2 Hz, 1H), 7.32-7.36 (m, 2H), 7.61 (dd, J=0.4, 8.4 Hz, 1H), 8.04 (s, 1H).

### Production Example 5

### 7-Allyloxy-2, 2-dimethylchroman-4-one

9.74 g of 7-hydroxy-2,2-dimethylchroman-4-one (CAS#: 17771-33-4) was dissolved in 150 mL of *N,N-*dimethylformamide. To the reaction solution was added 10.5 g of potassium carbonate and 7.36 g of allyl bromide, and then the reaction solution was stirred at room temperature overnight. The reaction solution was diluted with ethyl acetate, and then washed with water and brine. The organic layer was dried over magnesium sulfate and then concentrated under reduced pressure. The resulting residue was then purified by silica gel column chromatography (hexane-ethyl acetate) to obtain 11.0 g of the title compound.
¹H-NMR (400 MHz, CDCl₃) δ (ppm): 1.45 (s, 6H), 2.67 (s, 2H), 4.53-4.58 (m, 2H), 5.28-5.35 (m, 1H), 5.37-5.46 (m, 1H), 5.98-6.09 (m, 1H), 6.38 (d, J=2.4 Hz, 1H), 6.56 (dd, J=2.4, 8.8 Hz, 1H), 7.80 (d, J=8.8 Hz, 1H).

### Production Example 6

### 8-Allyl-7-hydroxy-2,2-dimethylchroman-4-one

Under a nitrogen atmosphere, 1.97 g of 7-allyloxy-2,2-dimethylchroman-4-one was dissolved in 5 mL of N,N-dimethylaniline, and the resultant reaction solution was heated to reflux for 6 hours. The reaction solution was allowed to cool to room temperature, and was then purified by silica gel column chromatography (hexane-ethyl acetate) to obtain the title compound. The obtained compound was subjected to further purification by high performance liquid chromatography (ODS-AM; acetonitrile-water) to obtain 1.05 g of the title compound.
¹H-NMR (400 MHz, CDCl₃) δ (ppm): 1.44 (s, 6H), 2.66 (s, 2H), 3.40-3.46 (m, 2H), 5.03-5.17 (m, 2H), 5.55 (s, 1H), 5.86-6.00 (m, 1H), 6.47 (d, J=8.8 Hz, 1H), 7.71 (d, J=8.8 Hz, 1H).

### Production Example 7

### 8-Allyl-7-methoxy-2,2-dimethylchroman-4-one

567 mg of 8-allyl-7-hydroxy-2,2-dimethylchroman-4-one was dissolved in 15 mL of *N,N-*dimethylformamide. To the reaction solution was added 0.51 g of potassium carbonate and 0.42 g of iodomethane, and then the reaction solution was stirred at room temperature overnight. The reaction solution was diluted with ethyl acetate, and then washed with saturated aqueous ammonium chloride and brine. The organic layer was dried over magnesium sulfate and then concentrated under reduced pressure. The resulting residue was purified by silica gel column chromatography (hexane-ethyl acetate) to obtain 582 mg of the title compound.
¹H-NMR (400 MHz, CDCl₃) δ (ppm): 1.44 (s, 6H), 2.67 (s, 2H), 3.36-3.40 (m, 2H), 3.88 (s, 3H), 4.92-5.04 (m, 2H), 5.84-5.95 (m, 1H), 6.58 (d, J=8.8 Hz, 1H), 7.80 (d, J=8.8 Hz, 1H).

### Production Example 8

### Production of 1-{1-[2-(7-methoxy-2,2-dimethyl-4-oxochroman-8-yl)ethyl]piperidin-4-yl}-N-methyl-1H-indole-6-carboxamide

Under a nitrogen atmosphere, 126 mg of 8-allyl-7-methoxy-2,2-dimethylchroman-4-one was dissolved in 12 mL of *t*-butanol-water (1:1). To the resultant reaction solution was added 0.72 g of AD-mix-β and then the reaction mixture was stirred at room temperature for 24 hours. Under ice-cooling, 0.77 g of sodium sulfite was added to the reaction solution and then the reaction mixture was stirred at room temperature for 1 hour. The reaction mixture was diluted with ethyl acetate and then washed with brine. The organic layer was dried over magnesium sulfate, filtered and then concentrated under reduced pressure to obtain 145 mg of 8-(2,3-dihydroxypropyl)-7-methoxy-2,2-dimethylchroman-4-one. This compound was used in the following reaction without any further purification.
145 mg of 8-(2,3-dihydroxypropyl)-7-methoxy-2,2-dimethylchroman-4-one was dissolved in 3 mL of tetrahydrofuran and 4 mL of methanol. A solution of 0.22 g of sodium metaperiodate in 7 mL of water was added to the resultant reaction solution under ice-cooling and then the reaction mixture was stirred at room temperature for 30 minutes. The reaction mixture was diluted with ethyl acetate and then washed with brine. The organic layer was dried over magnesium sulfate, filtered and then concentrated under reduced pressure to obtain 120 mg of (7-methoxy-2,2-dimethyl-4-oxochroman-8-yl)acetaldehyde. This compound was used in the following reaction without any further purification.
120 mg of *N*-methyl-1-(piperidin-4-yl)-1*H-*indole-6-carboxamide and 120 mg of (7-methoxy-2,2-dimethyl-4-oxochroman-8-yl)acetaldehyde were dissolved in 8 mL of methylene chloride. To the resultant reaction solution was added 0.05 mL of acetic acid and 0.15 g of sodium triacetoxyborohydride, and then the reaction mixture was stirred at room temperature for 1 hour. A saturated aqueous sodium bicarbonate was added to the reaction mixture and then the mixture was extracted with methylene chloride. The extract was dried over magnesium sulfate, filtered and then concentrated under reduced pressure. The resulting residue was purified by silica gel column chromatography (methanol-ethyl acetate) to obtain 210 mg of the title compound.
¹H-NMR (400 MHz, DMSO-d₆) δ (ppm): 1.40 (s, 6H), 1.92-2.10 (m, 4H), 2.22-2.33 (m, 2H), 2.40-2.50 (m, 2H), 2.72 (s, 2H), 2.74-2.83 (m, 2H), 2.82 (d, J=4.4 Hz, 3H), 3.08-3.17 (m, 2H), 3.87 (s, 3H), 4.35-4.47 (m, 1H), 6.50 (d, J=3.2 Hz, 1H), 6.75 (d, J=9.2 Hz, 1H), 7.51-7.59 (m, 2H), 7.62-7.69 (m, 2H), 8.06 (s, 1H), 8.29-8.37 (m, 1H).

### Production Example 9

### Production of 1-{1-[2-(7-methoxy-2,2-dimethyl-4-oxochroman-8-yl)ethyl]piperidin-4-yl}-N-methyl-1H-indole-6-carboxamide fumarate

1.00 g of 1-{1-[2-(7-Methoxy-2,2-dimethyl-4-oxochroman-8-yl)ethyl]piperidin-4-yl}-*N*-methyl-1*H-*indole-6-carboxamide and 0.249 g of fumaric acid were dissolved in a mixed solvent of 5 mL of acetone and 15 mL of water at 60°C. The resultant reaction solution was then left at room temperature for 1 hour. The precipitated solid was collected by filtration and then washed with a mixed solvent of 2.5 mL of acetone and 7.5 mL of water to obtain the 1.09 g of the title compound.
¹H-NMR (400 MHz, DMSO-d₆) δ (ppm): 1.40 (s, 6H), 1.94-2.11 (m, 4H), 2.27-2.37 (m, 2H), 2.45-2.56 (m, 2H), 2.72 (s, 2H), 2.75-2.84 (m, 5H), 3.12-3.20 (m, 2H), 3.87 (s, 3H), 4.38-4.47 (m, 1H), 6.48-6.51 (m, 1H), 6.60 (s, 1.5H), 6.75 (d, J=9.6 Hz, 1H), 7.50-7.58 (m, 2H), 7.63-7.67 (m, 2H), 8.05 (brs, 1H), 8.29-8.35 (m, 1H).

### Production Example 10

### Synthesis of 1-{1-[2-(7-methoxy-2,2-dimethyl-4-oxochroman-8-yl)ethyl]piperidin-4-yl}-N-methyl-1H-indole-6-carboxamide L-(+)-tartrate

100 mg of 1-{1-[2-(7-Methoxy-2,2-dimethyl-4-oxochroman-8-yl)ethyl]piperidin-4-yl}-*N*-methyl-1*H-*indole-6-carboxamide was dissolved in a mixed solvent of 1 mL of tetrahydrofuran and 25 mL of diethyl ether. To the resultant reaction solution was added at room temperature 31 mg of L-(+)-tartaric acid in a mixed solvent of 1 mL of tetrahydrofuran and 25 mL of diethyl ether. The precipitated solid was collected by filtration and then washed with diethyl ether to obtain 110 g of the title compound.
¹H-NMR (400 MHz, DMSO-d₆) δ (ppm): 1.40 (s, 6H), 1.97-2.14 (m, 4H), 2.40-2.60 (m, 4H), 2.72 (s, 2H), 2.78-2.84 (m, 5H), 3.20-3.30 (m, 2H), 3.87 (s, 3H), 4.20 (s, 2H), 4.43-4.53 (m, 1H), 6.50 (d, J=3.2 Hz, 1H), 6.75 (d, J=8.4 Hz, 1H), 7.50-7.58 (m, 2H), 7.63-7.67 (m, 2H), 8.05 (br s, 1H), 8.28-8.34 (m, 1H).

### Example 1

### Synthesis of 1-{1-[2-(7-methoxy-2,2-dimethyl-4-oxochroman-8-yl)ethyl]piperidin-4-yl}-N-methyl-1H-indole-6-carboxamide fumarate (form A crystal)

1.00 g of 1-{1-[2-(7-methoxy-2,2-dimethyl-4-oxochroman-8-yl)ethyl]piperidin-4-yl}-*N*-methyl-1*H-*indole-6-carboxamide and 0.249 g of fumaric acid were dissolved in a mixed solvent of 5 mL of acetone and 15 mL of water at 60°C, and the resultant reaction solution was then left at room temperature for 1 hour. The precipitated solid was collected by filtration, washed with a mixed solvent of 2.5 mL of acetone and 7.5 mL of water to obtain 1.09 g of the title compound.

### Example 2

### Synthesis of 1-{1-[2-(7-methoxy-2,2-dimethyl-4-oxochroman-8-yl)ethyl]piperidin-4-yl}-N-methyl-1H-indole-6-carboxamide fumarate (form B crystal)

2.05 g of 1-{1-[2-(7-methoxy-2,2-dimethyl-4-oxochroman-8-yl)ethyl]piperidin-4-yl}-*N*-methyl-1*H-*indole-6-carboxamide fumarate was dissolved in a mixed solvent of 6 mL of *n*-propanol and 18 mL of water at 60°C, and the resultant reaction solution was left at room temperature and then at 0°C. The precipitated crystals were collected by filtration and then dried at room temperature under reduced pressure for 30 minutes to obtain 2.02 g of the title compound.

### Example 3

### Synthesis of 1-{1-[2-(7-methoxy-2,2-dimethyl-4-oxochroman-8-yl)ethyl]piperidin-4-yl}-N-methyl-1H-indole-6-carboxamide fumarate (form C crystal)

100 mg of 1-(1-[2-(7-methoxy-2,2-dimethyl-4-oxochroman-8-yl)ethyl]piperidin-4-yl}-*N*-methyl-1*H-*indole-6-carboxamide fumarate was weighed and placed in a round-bottom flask. The compound was once dissolved in a mixed solvent of 1 mL of water and 0.6 mL of methanol under heating conditions, and the resultant reaction solution was then left at room temperature. The precipitated crystals were collected by filtration and then dried at 60°C to obtain 68 mg of the title compound.

### Example 4

### Synthesis of 1-{1-[2-(7-methoxy-2,2-dimethyl-4-oxochroman-8-yl)ethyl]piperidin-4-yl}-N-methyl-1H-indole-6-carboxamide fumarate (form D crystal)

100 mg of 1-{1-[2-(7-methoxy-2,2-dimethyl-4-oxochroman-8-yl)ethyl]piperidin-4-yl}-*N*-methyl-1*H-*indole-6-carboxamide fumarate was weighed and placed in a round-bottom flask. The compound was once dissolved in 1 mL of 2-propanol under heating conditions, and the resultant reaction solution was then left at room temperature. The precipitated crystals were collected by filtration and then dried at 60°C to obtain 80 mg of the title compound.

### Example 5

### Synthesis of 1-{1-[2-(7-methoxy-2,2-dimethyl-4-oxochroman-8-yl)ethyl]piperidin-4-yl}-N-methyl-1H-indole-6-carboxamide fumarate (form D crystal) (Separate Process)

1,322.8 g of brown, oily 1-{1-[2-(7-methoxy-2,2-dimethyl-4-oxochroman-8-yl)ethyl]piperidin-4-yl}-*N-*methyl-1*H*-indole-6-carboxamide (content 500.0 g) was dissolved by adding 427.2 mL of ethanol and 500 mL of 2-propanol. This solution was filtered through a filter paper and the filter paper was rinsed with 570 mL of ethanol to prepare a solution of 1-{1-[2-(7-methoxy-2,2-dimethyl-4-oxochroman-8-yl)ethyl]piperidin-4-yl}-*N*-methyl-1*H*-indole-6-carboxamide in ethanol/2-propanol.
A 10 L, four-necked, round-bottom flask was placed under a stream of nitrogen with 127.0 g of fumaric acid (1.05 mole equivalent, 98% content percentage), 1,000 mL of ethanol and 1,500 mL of 2-propanol. The solution was dissolved by heating to an external temperature of 75°C. To this fumaric acid solution was added dropwise over about 1 hour the solution of 1-{1-[2-(7-methoxy-2,2-dimethyl-4-oxochroman-8-yl)ethyl]piperidin-4-yl}-*N*-methyl-1*H-*indole-6-carboxamide in ethanol/2-propanol. The vessel which contained the solution of 1-{1-[2-(7-methoxy-2,2-dimethyl-4-oxochroman-8-yl)ethyl]piperidin-4-yl}-N-methyl-1*H*-indole-6-carboxamide in ethanol/2-propanol and the dropwise addition funnel were washed with 250 mL of ethanol. The temperature of the hot bath was lowered, 500 mg of seed crystals were added into the solution at 50 to 55°C, and then the solution was stirred overnight with slow cooling (temperature decreased to 21.6°C). The precipitated crystals were collected by filtration and washed with a mixed solution of ethanol/2-propanol (500 mL/500 mL). The crystals were then dried under reduced pressure at 40°C until a constant weight was reached to obtain 519.8 g of the title compound as pale yellow-white crystals (yield 84.0%).

### Example 6

### Synthesis of 1-{1-[2-(7-methoxy-2,2-dimethyl-4-oxochroman-8-yl)ethyl]piperidin-4-yl}-N-methyl-1H-indole-6-carboxamide crystalline of tartrates

To 654 g of 1-{1-[2-(7-methoxy-2,2-dimethyl-4-oxochroman-8-yl)ethyl]piperidin-4-yl}-*N*-methyl-1*H-*indole-6-carboxamide and 201 mg of tartaric acid were added 4 mL of 2-propanol and 10 mL of methanol, and the resultant mixture was dissolved by heating to about 50°C. The solution was concentrated under reduced pressure to obtain a tartrate. 40 mL of an 80% aqueous methanol was added into 80 mg of the tartrate to prepare a solution having a concentration of 2 mg/mL. Solvent was evaporated off under a stream of nitrogen to obtain 70 mg of crystalline of tartrates.

### Example 7

### Synthesis of 1-{1-[2-(7-methoxy-2,2-dimethyl-4-oxochroman-8-yl)ethyl]piperidin-4-yl}-N-methyl-1H-indole-6-carboxamide 1/2 L-(+)-crystalline of tartrates

3.15 g of 1-{1-[2-(7-methoxy-2,2-dimethyl-4-oxochroman-8-yl)ethyl]piperidin-4-yl}-*N*-methyl-1*H-*indole-6-carboxamide fumarate was added with 30 mL of methanol, 130 mL of ethyl acetate, 25 mL of 2 N sodium hydroxide and 60 mL of brine, and the resultant mixture was separated. The organic layer was washed with 60 mL of brine and then dried over 6 g of anhydrous magnesium sulfate. After removing the anhydrous magnesium sulfate by filtration, the filtrate was concentrated under reduced pressure to obtain 2.61 g of a free substance as a yellow-white amorphous. To 2.61 of the free substance was added 555 mg of L-(+)-tartrate and 35 mL of methanol, and the resultant mixture was dissolved by heating at about 50°C. This reaction solution was concentrated under reduced pressure to obtain tartrate in an amorphous form. To the tartrate was added 20 mL of methanol and 20 mL of water and the resultant solution was heated. The precipitate was filtered off from the reaction solution. The filtrate was concentrated under reduced pressure to obtain a residue. 18 mL of methanol and 20 mL of water was added to this residue and the resultant mixture was dissolved by heating at 60°C. The resultant reaction solution was then stirred while slowly cooling. 2 mL of water was added to the reaction solution and the stirring was continued. Once the precipitation of crystals had been observed, the stirring was stopped and the reaction solution was left to stand. The precipitated solids were collected by filtration and dried at 60°C for 3 hours to obtain 2.17 g of the title compound.

The (7-methoxy-2,2-dimethyl-4-oxochroman-8-yl)acetaldehyde which is obtained as an intermediate in Production Example 8 may also be prepared according to the following Reference Examples 1 to 7.

### Reference Example 1

### Synthesis of ethyl [2-(1-ethoxyethoxy)-6-methoxyphenyl]acetate

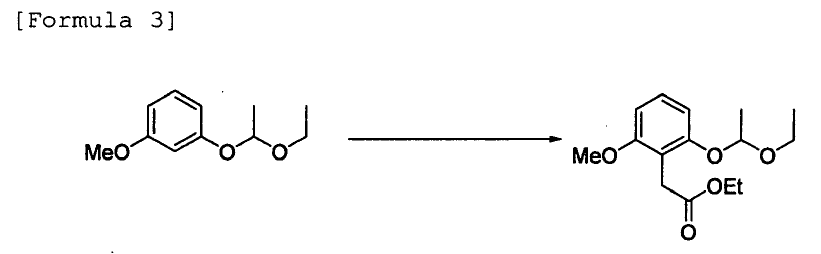

854.0 g of 1-(1-ethoxyethoxy)-3-methoxybenzene (content: 717.4 g, 3.656 mol) was placed in a 20 L reactor under a nitrogen atmosphere, rinsed with 7,174 mL of tetrahydrofuran and the resultant solution was then stirred. Coolant set at a temperature of 4°C was circulated in the jacket of the reactor. 1,156 g of *n*-butyllithium (4.414 mol, 2.71 M, *n*-hexane solution) was added dropwise over 41 minutes to the reaction solution. The reaction solution was then stirred at the same temperature for about 1.5 hours. The coolant temperature was set to -20°C, and once it had been confirmed that the internal temperature had reached -10°C or lower, 417.8 g (2.194 mol) of copper(I) iodide was added into the reaction solution over three stages. The reaction solution was then stirred at the same temperature for about 14 hours. The coolant set temperature was changed to - 90°C, and 702.1 g (4.204 mol) of ethyl bromoacetate was added dropwise over 26 minutes to the reaction solution. The resultant solution was then washed with 10 mL of tetrahydrofuran. After the dropwise addition was completed, the reaction solution was stirred for 44 minutes. The coolant set temperature was changed to - 35°C, and the reaction solution was stirred for further about 1.8 hours. The coolant set temperature was changed to -20°C, and after the internal temperature exceeded -20°C, the solution was stirred for 1 hour. The progress of the reaction was confirmed by HPLC. The reaction solution was added over about 30 minutes at the same temperature with 1,435 mL of 28% ammonia water, and the coolant temperature was changed to 25°C. 7,174 mL of toluene was added into the reaction solution for extraction, and the organic layer was successively washed with 1,440 mL of 28% ammonia water and tap water (3 times: 1,435 mL × 3). 127 mL (0.731 mol) of N,N-diisopropylethylamine was added to the resultant organic layer, and the resultant mixture was concentrated under reduced pressure to obtain a pale orange oil containing the title compound. Yield amount: 1,122.3 g; Content: 990.7 g; Yield percentage: 96.0%; HPLC purity: 70.6%
¹H-NMR (400 MHz, CDCl₃) δ (ppm): 1.19 (t, J=7.2 Hz, 3H), 1.24 (d, J=7.2 Hz, 3H), 1.47 (d, J=5.2 Hz, 3H), 3.46-3.56 (m, 1H), 3.66-3.82 (m, 3H), 3.80 (s, 3H), 4.14 (q, J=7.2 Hz, 2H), 5.39 (q, J=5.2 Hz, 1H), 6. 57 (d, J=8.4 Hz, 1H), 6.70 (d, J=8.8 Hz, 1H), 7.17 (dd, J=8.8, 8.4 Hz, 1H).

The synthesis methods represented by the following reaction scheme are illustrated in the following Reference Examples 2 to 4.

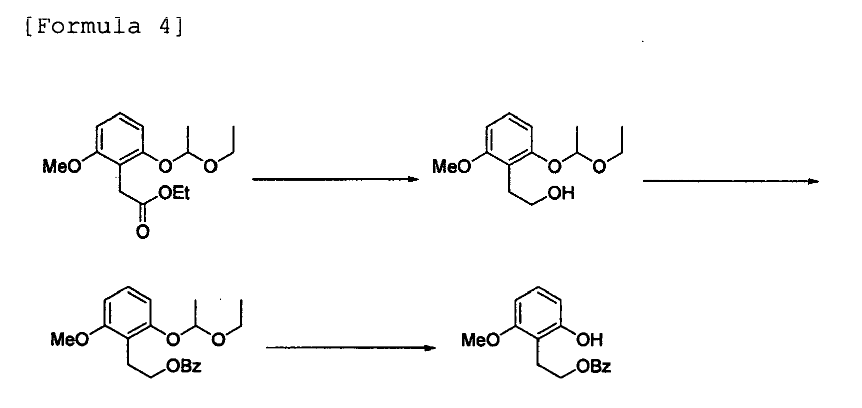

### Reference Example 2

### Synthesis of 2-[2-(1-ethoxyethoxy)-6-methoxyphenyl]ethanol

248.8 g of ethyl[2-(1-ethoxyethoxy)-6-methoxyphenyl] acetate (content: 213.0 g, 0.754 mol), 561.6 g of the same compound (content: 495.7 g, 1.756 mol), 8,504 mL of toluene and 2,126 mL of 1,2-dimethoxyethanewas successively added to a 15 L, four-necked, round-bottom flask under a nitrogen atmosphere, and stirring was started and the reaction vessel was cooled with ice. 1,403.7 g of sodium bis(2-methoxyethoxy)aluminum hydride (65% toluene solution, 1.8 mole equivalent) was added dropwise over 50 minutes to the reaction solution. Immediately after the dropwise addition was completed, the ice-water bath was changed to a water bath, and the reaction solution was stirred for 2.5 hours. The water bath was changed to an ice-water bath, and about 1.5 L of 8% (w/w) aqueous sodium hydroxide (prepared by charging 4,570 mL of water into 430 g of 93.0% sodium hydroxide) was added dropwise over 47 minutes to the reaction solution. The reaction solution was then transferred to a 20 L separatory funnel. All of the remaining prepared aqueous sodium hydroxide was added into the funnel, and the aqueous layer was discarded. The organic layer was washed three times (1,417 mL × 2, 709 mL × 1) with tap water, and then concentrated under reduced pressure (40°C). The title compound contained in the concentrated residue was assayed. Weight of concentrated residue: 1,042.0 g; Content: 563.3 g
¹H-NMR (400 MHz, CDCl₃) δ (ppm): 1.20 (t, J=7.2 Hz, 3H), 1.50 (d, J=5.6 Hz, 3H), 3.00 (t, J=6.8 Hz, 2H), 3.48-3.58 (m, 1H), 3.68-3.90 (m, 3H), 3.82 (s, 3H), 5.42 (q, J=5.6 Hz, 1H), 6.58 (d, J=8.0 Hz, 1H), 6.70 (d, J=8.4 Hz, 1H), 7.13 (dd, J=8.4, 8.0 Hz, 1H).

### Reference Example 3

### Synthesis of 2-[2-(1-ethoxyethoxy)-6-methoxyphenyl]ethyl benzoate

1,042.0 g of the concentrated residue of the organic layer obtained in Reference Example 2 was transferred to a 15 L, four-necked, round-bottom flask under a nitrogen atmosphere, and 8,102 mL of toluene, 2,025 mL of DME, 304.8 g of triethylamine and 29.2 g of N,N,N,N-tetramethylethyleneamine were successively added thereto. While stirring under ice-cooling, 388.1 g (2.761 mol) of benzoyl chloride was added dropwise over 40 minutes to the solution. The reaction solution was stirred at the same temperature for 10 minutes, the ice bath was then changed to a water bath, and the solution was stirred for a further 2.8 hours. The reaction solution was transferred to a 20 L separatory funnel and washed twice with 3,544 mL and 709 mL of tap water. The title compound contained in the 10.84 L of resultant organic layer was assayed.
Content: 745.0 g
¹H-NMR (400 MHz, CDCl₃) δ (ppm): 1.18 (t, J=7.2 Hz, 3H), 1.48 (d, J=5.2 Hz, 3H), 3.17 (t, J=7.2 Hz, 2H), 3.45-3.56 (m, 1H), 3.66-3.80 (m, 1H), 3.76 (s, 3H), 4.45 (t, J=7.2 Hz, 2H), 5.41 (q, J=5.2 Hz, 1H), 6.55 (d, J=8.4 Hz, 1H), 6.71 (d, J=8.0 Hz, 1H), 7.13 (dd, J=8.4, 8.0 Hz, 1H), 7.36-7.44 (m, 2H), 7.50-7.56 (m, 1H), 7.98-8.06 (m, 2H).

### Reference Example 4

### Synthesis of 2-(2-hydroxy-6-methoxyphenyl)ethyl benzoate

The organic layer obtained in Reference Example 3 was transferred to a 15 L, four-necked, round-bottom flask, and 2,126 mL of tetrahydrofuran was added thereto. The resultant solution was stirred while cooling with an ice-water bath. 1,417 mL of 5 N hydrochloric acid was added dropwise over 23 minutes to the solution. The solution was stirred at the same temperature for about 1 hour, then the chilled water in the bath was removed, and the stirring was continued for 2.5 hours. The reaction solution was transferred to a 20 L separatory funnel and the aqueous layer was discarded. The organic layer was washed with 8% aqueous sodium bicarbonate (prepared by charging 1,956 mL of water into 170 g of sodium bicarbonate) and twice with tap water (709 mL × 2). The resultant organic layer was concentrated under reduced pressure at a bath temperature of 40°C to obtain 1,463.0 g of slurry.
The obtained slurry was washed with 709 mL of tetrahydrofuran in a 10 L, four-necked, round-bottom flask. While stirring, 5,670 mL of a toluene-heptane mixed solution (1:8) was added dropwise over about 2.5 hours, and the resultant solution was stirred for about a further 14 hours at room temperature. The precipitated crystals were collected by filtration, washed with 708 mL of toluene-heptane mixed solution (1:8), dried for about 4.5 hours at a bath temperature of 40°C under reduced pressure to obtain the title compound as white crystals.
Yield amount: 535.9 g; Yield percentage: 78.4%
¹H-NMR (400 MHz, CDCl₃) δ (ppm): 3.15 (t, J=7.2 Hz, 2H), 3.79 (s, 3H), 4.45 (t, J=7.2 Hz, 2H), 5.86 (s, 1H), 6.48 (d, J=8.4 Hz, 1H), 6.53 (d, J=8.4 Hz, 1H), 7.09 (dd, J=8.4, 8.4 Hz, 1H), 7.44 (dd, J=7.6, 7.6 Hz, 2H), 7.56 (dd, J=7.6, 7.6 Hz, 1H), 8.04 (d, J=7.6 Hz, 1H).

### Reference Example 5

### Synthesis of 2-(7-methoxy-2,2-dimethyl-4-oxochroman-8-yl)ethyl benzoate

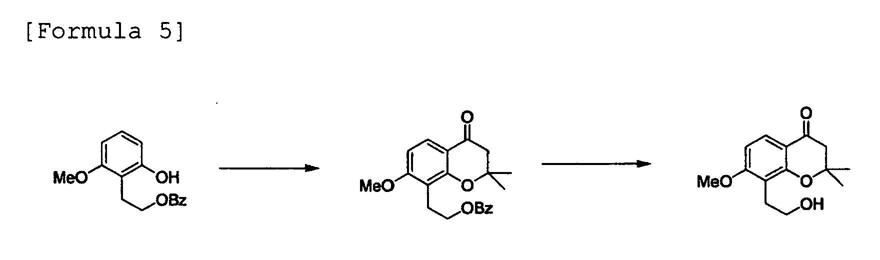

202.2 g (2.020 mol) of 3-methylcrontonic acid and 2 L of methanesulfonic acid was placed in a 10 L, four-necked, round-bottom flask , and the resultant solution was stirred in a stream of nitrogen on a water bath having a temperature of 50°C. This solution was added with 500.0 g (1.836 mol) of the 2-(2-hydroxy-6-methoxyphenyl)ethyl benzoate obtained in Reference Example 4. The resultant reaction mixture was stirred for 1.8 hours at the same temperature, and then cooled with ice. 2.5 L of toluene was added to the reaction solution, and then 5 L of tap water was added dropwise over about 1 hour. The contents were transferred to a 20 L separatory funnel, and the aqueous layer was discarded. The organic layer was washed three times with tap water (5 L × 3), and then concentrated under reduced pressure (bath temperature of 40°C) to obtain 846.1 g of the title compound as a brown oil.
¹H-NMR (400 MHz, CDCl₃) δ (ppm): 1.39 (s, 6H), 2.62 (s, 2H), 3.13 (t, J=6.8 Hz, 2H), 3.82 (s, 3H), 4.45 (t, J=6.8 Hz, 2H), 6.57 (d, J=8.8 Hz, 1H), 7.38-7.45 (m, 2H), 7.51-7.57 (m, 1H), 7.82 (d, J=8.8 Hz, 1H), 7.98-8.04 (m, 2H).

### Reference Example 6

### Synthesis of 8-(2-hydroxyethyl)-7-methoxy-2,2-dimethylchroman-4-one

844.9 g of oil obtained in Reference Example 5 was transferred to a 20 L, four-necked, round-bottom flask using 2.5 L of tetrahydrofuran. 2.5 L of methanol was added to the solution in tetrahydrofuran and the resultant solution was cooled with water (water temperature of 22°C). Under stirring, 8% (w/w) aqueous sodium hydroxide (prepared by charging 1,678 mL of water into 158 g of sodium hydroxide (93.0%)) was added dropwise over 18 minutes to the solution. After the dropwise addition was completed, the water bath was removed, and the reaction solution was stirred for about 3.5 hours at room temperature. 10 L of tap water was added dropwise over about 1 hour to the reaction solution. The reaction vessel was cooled with ice, and the reaction solution was stirred for about 1 hour at an internal temperature of 10°C or less. The precipitated crystals were collected by filtration and successively washed with 2 L of tap water and 2 L of a methanol-tap water mixture (1:4). The obtained crystals were then dried under reduced pressure at 40°C until the weight became constant to obtain 374.7 g of crude title compound as a pale yellow-white solid. Yield amount: 374.7 g; Content: 305.8 g; Yield percentage: 66.6%; HPLC purity: 84.5%
A 15 L, four-necked, round-bottom flask was added with 374.7 g (content 305.8 g) of the crude title compound and 2 L of ethyl acetate, and stirring was started while heating with a water bath heated to 80°C. The suspension was added with a further 4.1 L of ethyl acetate, and the bath temperature setting was changed to 75°C. After dissolution of the crystals was confirmed, the temperature of the water bath was slowly lowered, and seed crystals were added at an internal temperature of 45.3°C. Crystal precipitation was observed 6 minutes after the seed crystals were added. The temperature of the water bath was further lowered, and 6.116 L of heptane was added at an internal temperature of 30°C or less into the suspension over about 1 hour. The reaction solution was stirred for about 13 hours at the same temperature. The suspension was cooled with ice and stirred for about 4 hours. The crystals were then collected by filtration using a Buchner funnel, and washed with 918 mL of a mixed solution of ethyl acetate-heptane (1:2). The obtained crystals were dried under reduced pressure for about 3 hours on a water bath having a temperature of 40°C, and then dried under reduced pressure for about 14 hours at room temperature to obtain the title compound as a grayish white solid.
Yield amount: 294.5 g; Content: 275.4 g; Yield percentage: 90.1%; HPLC purity: 98.7%
¹H-NMR (400 MHz, CDCl₃) δ (ppm): 1.45 (s, 6H), 2.68 (s, 2H), 2.96 (t, J=6.8 Hz, 2H), 3.73-3.80 (m, 2H), 3.89 (s, 3H), 6.59 (d, J=8.8 Hz, 1H), 7.81 (d, J=8.8 Hz, 1H).

### Reference Example 7

### Synthesis of (7-methoxy-2,2-dimethyl-4-oxochroman-8-yl)acetaldehyde

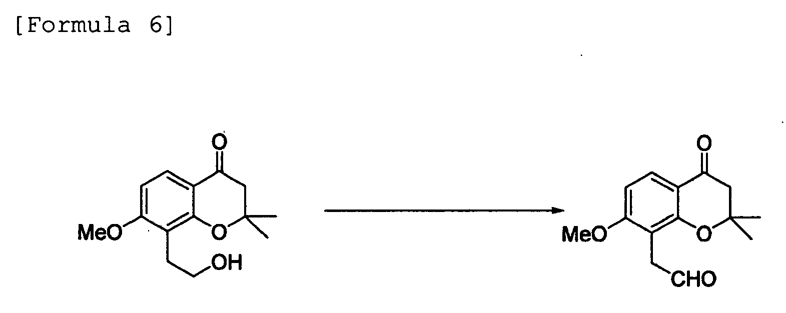

248.3 g (content 232.7 g, 0.930 mol) of 8-(2-hydroxyethyl)-7-methoxy-2,2-dimethylchroman-4-one, 294.0 g (content 274.9, 1.098 mol) of the same compound and 7,614 mL of ethyl acetate were placed in a 15 L, four-necked, round-bottom flask and the resultant solution was stirred. Cooling of this suspension was started using a cooling bath set at a temperature of - 4°C, and 161.9 g (1.574 mol) of sodium bromide , 508 mL of tap water and 3.17 g (20.28 mmol) of 2,2,6,6-tetramethylpiperidine oxide was successively added to the suspension. After the internal temperature reached 0°C, a mixed solution of 5.536 mol sodium hypochlorite solution and 2,538 g of 7% (w/w) aqueous sodium bicarbonate was added dropwise over about 2 hours into the flask. After the dropwise addition was completed, the temperature of the cooling bath was changed to 0°C, and the reaction solution was stirred for another 45 minutes. The reaction solution was transferred to a 20 L separatory funnel, and the aqueous layer was discarded. The organic layer was successively washed with 2,030 g of 10% aqueous sodium chloride and 2,030 mL of tap water. The resultant organic layer was concentrated under reduced pressure (40°C) to obtain 743.7 g of slurry. 500 mL of DME was added to the obtained slurry to form a solution. This solution was again concentrated under reduced pressure (40°C), and 500 mL of DME was again added to the precipitated crystals to dissolve. The solution was transferred to a 5 L, four-necked, round-bottom flask and then heated by a water bath having a temperature of 40°C. 515 mL of DME was further added to the reaction solution and stirred at 183 rpm. 500 mL of tap water was added to the solution was and cooling was started four minutes later with an ice-water solution. Seed crystals were added to the reaction solution and then stirred for about 1 hour. 515 mL of tap water was further added over about 30 minutes to the reaction solution and the mixture was stirred for another 1.3 hours. The reaction solution was then added over about 1 hour with 1,523 mL of heptane and stirred for about 1 hour or more at the same temperature. The precipitated crystals were collected by filtration, washed with a mixed solution of DME/tap water/heptane (using about 600 mL of a solution which mixed DME/tap water/heptane in a ratio of 1/1/1.5) and dried under reduced pressure (bath temperature of 40°C) until their mass was fairly constant to obtain the title compound as a yellowish white solid.
Yield amount: 478.0 g; Content: 413.9 g; Yield percentage: 82.2%; HPLC purity: 98.5%
¹H-NMR (400 MHz, CDCl₃) δ (ppm): 1.43 (s, 6H), 2.69 (s, 2H), 3.71 (s, 2H), 3.89 (s, 3H), 6.63 (d, J=8.8 Hz, 1H), 7.89 (d, J=8.8 Hz, 1H), 9.64 (s, 1H).

### Test Examples

The following tests were conducted to illustrate the usefulness of the compound represented by general formula (I) according to the present invention.

### Test Example 1

### Test Regarding Affinity for Rat Serotonin _{1A}

### Receptor

### (1) Method

An MPPF rat hippocampal membrane fraction selectively binding to the 5-HT_{1A} receptor was used to test affinity of a test substance for the rat 5-HT_{1A} receptor.
A rat hippocampus sample was homogenized in a 50 mM Tris-HCl buffer (pH 7.4; hereinafter referred to as "buffer A") that had been cooled with ice. The suspension was centrifuged at 50,000 × g for 20 minutes. The obtained sediment was suspended in buffer solution A, and the resultant solution was then centrifuged at 50,000 × g for 20 minutes. The obtained sediment was suspended in buffer solution to obtain a rat hippocampus membrane fraction.
The mixture used for incubation contained an appropriate amount of membrane fraction, a test substance at a desired concentration, [³H]MPPF, dimethyl sulfoxide and buffer A. The reaction was initiated through the addition of the membrane fraction, and the mixture was incubated at 25°C for 60 minutes. After incubation, the mixture was subjected to vacuum filtration by passing through a glass filter using a Cell Harvester. The filter was washed 3 times with buffer solution A, and then radioactivity binding to the receptor was measured with a liquid scintillation counter. Non-specific binding was defined as binding detected in the presence of 10 µM serotonin. The affinity data is shown in the following Table 13 as a Ki value calculated using the IC50 value determined from an inhibition curve, the used tracer concentration and the Kd value determined from Scatchard analysis.

### (2) Results

As can be seen from the results of Table 13, the fumarate compound according to the present invention exhibits excellent receptor binding activity.

### [Table 13]

**Table 13: Receptor binding activity**

| Test substance compound | Rat 5-HT_{1A} Ki(nM) |
|---|---|
| Form D crystal form of fumarate | 0.045 |

### Test Example 2

### Inhibitory Action Against the Increased Urinary Reflex Action Due to Destruction of the Superior Colliculus in Rats

### (1) Method

In the present test, Sprague-Dawley female rats (200-350 g) were used. The rats underwent a median incision of the abdomen under anesthesia. A hole with a minor diameter was made on the apex of the bladder, and a catheter used for measurement of an intravesical pressure was placed therein. A catheter used for administration of a test substance was placed in the femoral vein. These catheters were fixed at the occipital region of the rat through the subcutis. One day later, the urinary reflex of the rats was measured with a cystometrogram. Thereafter, the rats were fixed on a brain stereotaxis apparatus under anesthesia, and then subjected to a median incision of the scalp. Thereafter, a hole was created with a dental drill in the cranium at an upper portion of the superior colliculus in accordance with the coordinate of a brain diagram. A legion generator microelectrode (diameter: 0.7 mm; length: 1.5 mm) was then inserted into the superior colliculus through the hole. Electric current was then applied (65°C, 4 minutes) so as to damage the brain tissue. After completion of the operation, when the rat awoke from the anesthesia, cystometrogram was conducted again to confirm the increased state of urinary reflex. A test substance was administered through the catheter placed in the femoral vein, and the action of the test substance on urinary reflex was evaluated. In addition, the effects of several test substances were compared using the maximal reaction (Emax). The results are shown in Table 14.

### (2) Results

As can be seen from the results of Fig. 14, the fumarate compound according to the present invention exhibits an excellent pharmacological effect.

### [Table 14]

**[Table 14] Action against urinary reflex**

| Test substance compound | Administered amount (mg/kg, i.v.) | Urinary interval Emax (%) |
|---|---|---|
| Form D crystals of fumarate | 1 | 75 |

### Formulation Examples

Formulation examples for crystals of the compound according to the present invention will be described below. However, formulation of the crystals of the compound according to the present invention is not limited to these formulation examples.

### Formulation Example 1

Mixed uniformly together were 45 parts by weight of crystals of the compound synthesized in Working Example 1, 15 parts by weight of heavy magnesium oxide and 75 parts by weight of lactose so as to obtain a powder or fine granule powder with a size of 350 µm or less. This powder was encapsulated in a capsule container to produce a capsule.

### Formulation Example 2

Mixed uniformly together were 45 parts by weight of crystals of the compound synthesized in Working Example 5, 15 parts by weight of starch, 16 parts by weight of lactose, 21 parts by weight of crystalline cellulose, 3 parts by weight of polyvinyl alcohol and 30 parts by weight of distilled water. The resultant mixture was granulated by crushing and then dried. Thereafter, the resultant product was separated by sieving to obtain granules with a size between 1,410 and 177 µm.

### Formulation Example 3

Granules were produced in the same manner as in Formulation Example 2. Then, 4 parts by weight of calcium stearate were added to 96 parts by weight of these granules. The resultant granules were subjected to compression molding to produce a tablet with a diameter of 10 mm.

### Formulation Example 4

10 parts by weight of crystalline cellulose and 3 parts by weight of calcium stearate were added to 90 parts by weight of the granules obtained by the method described in Formulation Example 2. The resultant mixture was subjected to compression molding to produce a tablet with a diameter of 8 mm. Then, a mixed suspension containing syrup gelatin and precipitated calcium carbonate was added to the tablet to produce a sugarcoated tablet.

### Formulation Example 5

Mixed together and heated were 0.6 parts by weight of crystals of the compound synthesized in Working Example 2, 2.4 parts by weight of nonionic surfactant and 97 parts by weight of physiological saline solution. The resultant mixture was then placed in an ampule, which was sterilized so as to produce an injection.

### Formulation Example 6

Crystals of the compound synthesized in Working Example 1, lactose, corn starch and low-substituted hydroxypropyl cellulose were mixed together, and the resultant mixture was subjected to wet granulation using hydroxypropyl cellulose dissolved in an appropriate amount of purified water. The thus-granulated product was dried and then sized. Thereafter, low-substituted hydroxypropyl cellulose and magnesium stearate were added to the resultant granules, and these ingredients were then mixed and formed into a tablet. The obtained tablet was coated with an aqueous solution containing a coating base (opadry yellow). The amounts of raw materials used per tablet are shown in Table 15.

**[Table 15]**

| Used raw material | 1 mg tablet | 10 mg tablet | 60 mg tablet |
|---|---|---|---|
| Present Invention Compound 1 | mg | 10 mg | 60 mg |
| Lactose | 122 mg | 113 mg | 63 mg |
| Corn starch | 20 mg | 20 mg | 20 mg |
| Low-substituted hydroxypropyl cellulose | 20 mg | 20 mg | 20 mg |
| Hydroxypropyl cellulose | 6 mg | 6 mg | 6 mg |
| Distilled water Distilled | Appropriate amount | Appropriate amount | Appropriate amount |
| Low-substituted hydroxypropyl cellulose | 10 mg | 10 mg | 10 mg |
| Crystal cellulose | 20 mg | 20 mg | 20 mg |
| Magnesium stearate | 1 mg | 1 mg | 1 mg |
| Opadry yellow* | 8 mg | 8 mg | 8 mg |
| Total | 208 mg | 208 mg | 208 mg |

### INDUSTRIAL APPLICABILITY

A crystal of 1-{1-[2-(7-methoxy-2,2-dimethyl-4-oxochroman-8-yl)-ethyl]piperidin-4-yl}-*N*-methyl-1*H-*indole-6-carboxamide according to the present invention can be easily produced on an industrial scale free from metals or other such impurities and which is in single crystal form .

## Claims

1. A crystal form of 1-{1-[2-(7-methoxy-2,2-dimethyl-4-oxochroman-8-yl)-ethyl]piperidin-4-yl}-N-methyl-1*H*-indole-6-carboxamide fumarate.

2. A crystal form of 1-{1-[2-(7-methoxy-2,2-dimethyl-4-oxochroman-8-yl)-ethyl]piperidin-4-yl}-*N-*methyl-1*H*-indole-6-carboxamide fumarate which has peaks at chemical shifts of about 124.0 ppm and about 26.8 ppm in a ¹³C solid NMR spectrum.

3. A crystal form of 1-{1-[2-(7-methoxy-2,2-dimethyl-4-oxochroman-8-yl)-ethyl]piperidin-4-yl}-*N-*methyl-1*H*-indole-6-carboxamide fumarate which has peaks at chemical shifts of about 143.8 ppm and about 32.8 ppm in a ¹³C solid NMR spectrum.

4. A crystal form of 1-{1-[2-(7-methoxy-2,2-dimethyl-4-oxochroman-8-yl)-ethyl]piperidin-4-yl}-N-methyl-1*H*-indole-6-carboxamide fumarate which has peaks at chemical shifts of about 190.5 ppm and about 138.0 ppm in a ¹³C solid NMR spectrum.

5. A crystal form of 1-{1-[2-(7-methoxy-2,2-dimethyl-4-oxochroman-8-yl)-ethyl]piperidin-4-yl}-N-methyl-1*H*-indole-6-carboxamide fumarate which has diffraction peaks at diffraction angles (2θ±0.2°) of 18.2° and 30.9° in X-ray powder diffraction.

6. A crystal form of 1-{1-[2-(7-methoxy-2,2-dimethyl-4-oxochroman-8-yl)-ethyl]piperidin-4-yl}-N-methyl-1*H*-indole-6-carboxamide fumarate which has diffraction peaks at diffraction angles (2θ±0.2°) of 27.6° and 32.7° in X-ray powder diffraction.

7. A crystal form of 1-{1-[2-(7-methoxy-2,2-dimethyl-4-oxochroman-8-yl)-ethyl]piperidin-4-yl}-N-methyl-1*H*-indole-6-carboxamide fumarate which has diffraction peaks at diffraction angles (2θ±0.2°) of 9.8° and 19.7° in X-ray powder diffraction.

8. A crystal form of 1-{1-[2-(7-methoxy-2,2-dimethyl-4-oxochroman-8-yl)-ethyl]piperidin-4-yl}-N-methyl-1H-indole-6-carboxamide fumarate which has diffraction peaks at diffraction angles (2θ±0.2°) of 8.3° and 14.0° in X-ray powder diffraction .

9. A process for producing the crystal form according to claim 2 or 5, comprising heating 1-{1-[2-(7-methoxy-2,2-dimethyl-4-oxochroman-8-yl)-ethyl]piperidin-4-yl}-*N*-methyl-1*H*-indole-6-carboxamide fumarate in a mixed solvent of acetone and water to dissolve it, then cooling the solution to precipital crystals and filtering off the crystals.

10. A process for producing the crystal form according to claim 3 or 6, comprising heating 1-{1-[2-(7-methoxy-2,2-dimethyl-4-oxochroman-8-yl)-ethyl]piperidin-4-yl}-*N*-methyl-1*H*-indole-6-carboxamide fumarate in a mixed solvent of *n*-propanol and water to dissolve it, then cooling the solution to precipital crystals and filtering off the crystals.

11. A process for producing the crystal form according to claim 7, comprising heating 1-{1-[2-(7-methoxy-2,2-dimethyl-4-oxochroman-8-yl)-ethyl]piperidin-4-yl}-*N*-methyl-1*H*-indole-6-carboxamide fumarate in a mixed solvent of methanol and water to dissolve it, then cooling the solution to precipital crystals and filtering off the crystals.

12. A process for producing the crystal form according to claim 4 or 8, comprising heating 1-{1-[2-(7-methoxy-2,2-dimethyl-4-oxochroman-8-yl)-ethyl]piperidin-4-yl}-*N*-methyl-1*H*-indole-6-carboxamide fumarate in an alcohol solvent, an amide solvent, an ester solvent or a mixed solvent thereof to dissolve it, then cooling the solution to precipital crystals and filtering off the crystals.

13. A crystal form of 1-{1-[2-(7-methoxy-2,2-dimethyl-4-oxochroman-8-yl)-ethyl]piperidin-4-yl}-*N-*methyl-1*H*-indole-6-carboxamide tartrate.

14. A process for producing the crystal form according to claim 13, comprising dissolving 1-{1-[2-(7-methoxy-2,2-dimethyl-4-oxochroman-8-yl)-ethyl]piperidin-4-yl}-*N*-methyl-1*H*-indole-6-carboxamide tartrate in a mixed solvent of methanol and water, then distilling the mixed solvent.

15. A pharmaceutical composition comprising the crystal form according to any one of claims 1 to 8 and 13 as an active ingredient.

16. A preventive or therapeutic agent for lower urinary tract symptoms comprising the crystal form according to any one of claims 1 to 8 and 13 as an active ingredient.

17. The agent according to claim 16, which is A preventive or therapeutic agent for urinary storage symptoms.

18. The agent according to claim 16, which is A preventive or therapeutic agent for urinary frequency or urinary incontinence.

19. A preventive or therapeutic agent for cognitive impairment associated with Alzheimer's disease or senile dementia, learning or memory disorder, or anxiety disorder, comprising the crystal form according to any one of claims 1 to 8 and 13 as an active ingredient.

20. A preventive or therapeutic agent for schizophrenia, emotional disorder, alcohol and/or cocaine dependence, nicotine addiction or symptoms associated with smoking cessation, or visual attention disorder, comprising the crystal form according to any one of claims 1 to 8 and 13 as an active ingredient.

21. A preventive or therapeutic agent for sleep disorder, migraine, temperature instability, eating disorder, vomiting, gastrointestinal disorder, or sexual dysfunction, comprising the crystal form according to any one of claims 1 to 8 and 13 as an active ingredient.
